# EUROPEAN PATENT APPLICATION

(11) **EP 4 012 720 A1**
(43) Date of publication of application: **15.06.2022**
(21) Application number: 20851567.6
(22) Date of filing: 06.08.2020
(51) Int. Cl.: G16H 50/30, A61B 5/00, A61B 5/107

(54) **GENETIC TESTING METHOD FOR IMPLEMENTING SKIN CARE COUNSELING**

(30) Priority: 09.08.2019 JP 2019147470
(71) Applicant: Shiseido Company, Ltd., Chuo-ku Tokyo 104-0061 (JP)
(72) Inventor: AMANO, Satoshi, Tokyo 1040061 (JP); YOSHIKAWA, Tatsuya, Tokyo 1040061 (JP); YASUDA, Chie, Tokyo 1040061 (JP); WAKESHIMA, Nozomu, Tokyo 1040061 (JP)
(74) Representative: Cabinet Beau de Loménie
(86) International application number: PCT/JP2020/030258
(87) International publication number: WO 2021/029332

(57) **Abstract**

Since skin condition is significantly influenced by environmental factors associated with lifestyle habits, it has been difficult to forecast skin condition by means of SNP analysis. The purpose of the present invention is to determine one's congenital skin characteristics, which has been difficult with the prior art. The present invention provides a method for determining not only one's congenital skin characteristics but also future skin type by determining skin condition from genetic and environmental factors.

## Description

### TECHNICAL FIELD

The present invention relates to a skin evaluating method, a skin evaluation system and a control program for a skin condition evaluation system, which are based on genetic testing. Skin counseling can be provided based on the evaluation method, system and program of the invention.

### BACKGROUND ART

Examination of skin based on genetic SNP analysis has been proposed in recent years, and in particular the relationship between SNPs and skin quality is being examined using genes coding for proteins that are directly related to skin quality (PTLs 1 to 4). Methods have therefore been proposed for predicting skin properties and skin trouble based on the results of SNP analysis. In such types of research, SNPs are examined in genes that have been shown to directly contribute to skin quality including wrinkles and pigmented spots. Examples of such target genes include MC1R, MMP1, SOD2, GPX1 and ASIP. Due to the large variety of genes that affect skin properties, continuing research is being conducted on the relationship between skin properties and SNPs of a larger number of genes (PTL 1: International Patent Publication No. WO2018/101449).

### [CITATION LIST]

### [PATENT LITERATURE]

[PTL 1] International Patent Publication No. WO2018/101449

### SUMMARY OF INVENTION

### [TECHNICAL PROBLEM]

Skin examination based on SNP analysis indicates the type of skin with which one is born, but skin condition is known to be highly affected by environmental factors that are in turn associated with lifestyle habits. It has therefore been difficult to judge congenital skin characteristics based only on current skin conditions. Furthermore, since congenital skin characteristics is closely associated with future skin condition, it has been found that the precision of future skin condition prediction is low when made only based on genetic factors. The present invention has been invented for solving this problem, and its object is to provide a method of assessing congenital skin characteristics and a method of predicting future skin condition, based on both genetic factors and environmental factors.

### [SOLUTION TO PROBLEM]

The present inventors have conducted research and development on a method of assessing congenital skin characteristics and a method of predicting future skin condition by a combination of genetic factors determined by SNP analysis, and environmental factors including lifestyle habits, and have established a method of evaluating current or future skin condition.

The present invention is a skin evaluating method using individual SNP information and at least one item of environmental factor information selected from the group consisting of age, body height, body weight, BMI, ultraviolet exposure information and smoking information,
wherein SNP information and environmental factor information are weighted for contribution to skin condition, to determine the skin condition.

The present invention further relates to a skin evaluation system in which the skin evaluating method is carried out, and to a program and control method for controlling the skin evaluation system.

### [ADVANTAGEOUS EFFECTS OF INVENTION]

By combining SNP information with environmental factor information, it is possible to evaluate current or future skin condition more accurately than by a method of determining skin type based on genetic information alone as in the prior art.

### BRIEF DESCRIPTION OF DRAWINGS

[Fig. 1] Fig. 1A is a diagram schematically representing an information processing device comprising an input unit, a storage unit, a processing unit, a learning unit and an output unit. Fig. 1B is a diagram schematically representing a system in which a learning unit is present in an external learning device of an information processing device.
[Fig. 2] Fig. 2 is a schematic view showing the flow for creating a learning model that determines a skin condition score.
[Fig. 3] Fig. 3 is a graph showing classification of subjects into a high value group and low value group, the subjects being in a fixed ratio on the high value side and low value side, based on measured values for a skin condition (wrinkles).
[Fig. 4] Fig. 4 is a schematic view showing the flow for creating a learning model for assessing skin type.
[Fig. 5A] Fig. 5A is a schematic view showing flow for creation of a learning model for determining skin condition score, wherein a dataset is divided by a processing unit into a learning dataset and a validation dataset, and based on the divided learning dataset, a learning model for a learning unit is constructed and the constructed learning model is validated.
[Fig. 5B] Fig. 5B is a schematic view showing flow for creation of a learning model for assessing skin type, wherein a dataset is divided by a processing unit into a learning dataset and a validation dataset, and based on the divided learning dataset, a learning model for a learning unit is constructed and the constructed learning model is validated.
[Fig. 6A-B] Fig. 6A is a graph with an ROC curve representing the relationship between sensitivity and specificity, drawn for validation of a learning model for assessing skin type. Fig. 6B is a graph for creation of a coefficient of determination R2, drawn for validation of a learning model for determining skin condition score.
[Fig. 6C] Fig. 6C is a graph of SNP appearance frequency, created for validation of a learning model.
[Fig. 7] Fig. 7 is a schematic diagram showing flow for predicting future skin condition using a learning model for determining skin condition score.
[Fig. 8] Fig. 8 is a schematic view showing flow for assessing skin type using a learning model for assessing skin type.
[Fig. 9A] Fig. 9A is a graph showing change in future skin condition score, using a learning model for determining skin condition score.
[Fig. 9B] Fig. 9B shows change in prediction of skin condition score, as represented in an application with a slide bar for age, and selection of tanning countermeasures and smoking habit.
[Fig. 10] Fig. 10A is a graph showing skin type assessed using different learning models for assessing skin type. Fig. 10B is a sector graph showing evaluation based on skin types of both tendency for and tendency against the skin type.

### DESCRIPTION OF EMBODIMENTS

The present invention relates to a skin evaluating method which uses individual SNP information (herein also referred to as "genetic information") and environmental factor information (herein also referred to as "environmental information"). By combining genetic information and environmental information it is possible to more accurately evaluate current or future skin condition. Specifically, the skin evaluating method includes a step of determining skin condition by weighting the contribution of genetic information and environmental information to skin condition. Specifically, in weighting the contribution to skin condition, the relationship between genetic information/environmental information and previously measured skin condition values is applied in machine learning, and genetic information and environmental information are inputted into the learning model that has been weighted for contribution to a specific skin condition.

The method of the invention can be realized by a skin evaluation system comprising an input unit, a learning unit, a processing unit and an output unit. Another aspect of the invention relates to the skin evaluation system. Specifically, the skin evaluation system includes the following:
an input unit from which individual SNP information, and environmental factor information selected from the group consisting of age, body height, body weight, BMI, ultraviolet exposure information and smoking information, are inputted,
a learning unit which uses a learning model which has learned based on the relationship of SNP information and environmental factor information with skin condition, and which is weighted for contribution to skin condition, to determine information regarding skin condition,
a processing unit that processes the skin condition that has been determined by the learning unit, and
an output unit that outputs the processed skin condition. Another aspect of the invention relates to a program or control method for controlling the skin evaluation system of the invention.

The program for controlling the skin condition evaluation system of the invention is a control program for a skin evaluation system comprising a learning unit that sets information for skin condition via a learning model that has been weighted for contribution to skin condition using inputted SNP information and environmental factor information, an input unit, an output unit and a processing unit, the program including the following commands:
inputting individual SNP information and environmental factor information selected from the group consisting of age, body height, body weight, BMI, ultraviolet exposure information and smoking information, which has been inputted through the input unit, to the learning unit,
setting information for a determined skin condition via a learning model for inputted SNP information and environmental factor information which has been weighted for contribution to skin condition, at the learning unit,
processing information for the set skin condition to the processing unit, and
outputting the processed skin condition to the output unit.

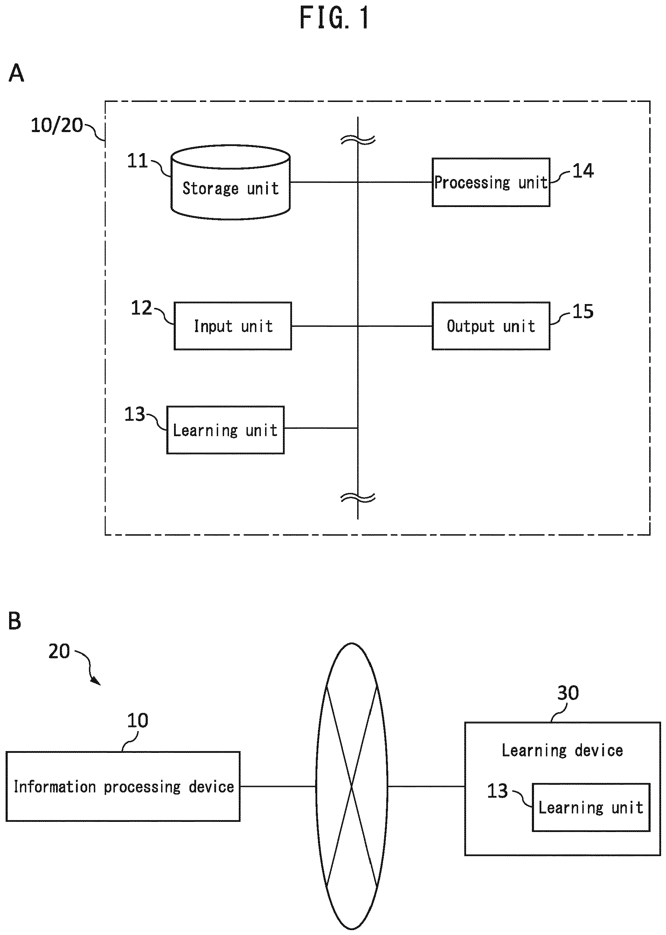

Fig. 1A is a diagram schematically representing an information processing device 10/system 20 comprising an input unit 12, a storage unit 11, a processing unit 14, a learning unit 13 and an output unit 15. The learning unit 13 is in the information processing device 10, but the learning unit 13 may instead be in an external learning device 30 that is connected with the information processing device 10 via a communication unit, forming the system 20 overall (Fig. 1B). The learning unit 13 that includes the created learning model may also be relocated to another information processing device. The connection between the external learning device 30 and information processing device 10 may be a wired or wireless connection. An example is connection via an intranet or the internet. The system of the present invention may also encompass devices.

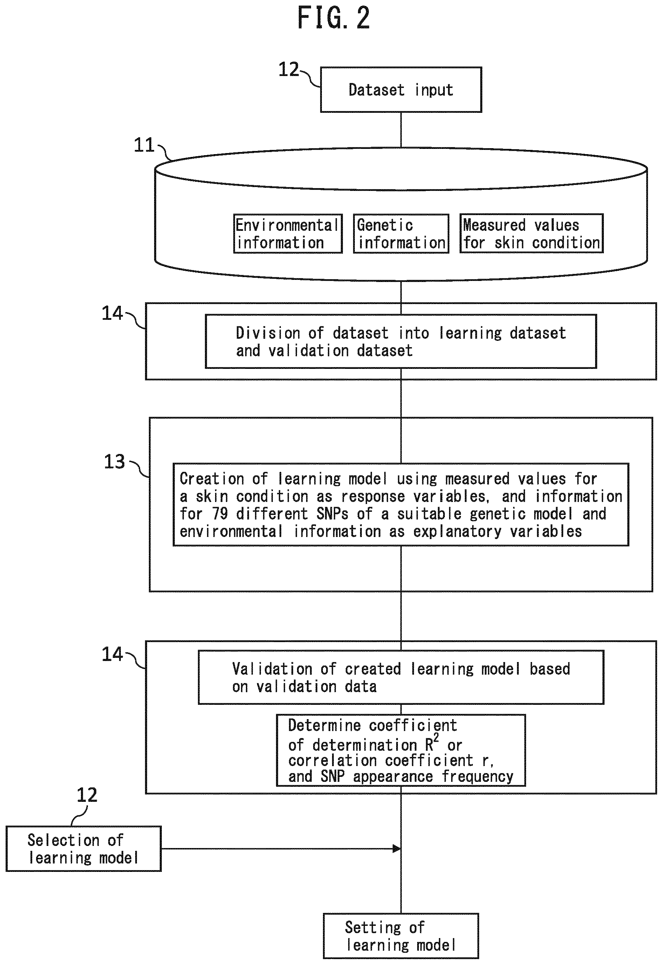

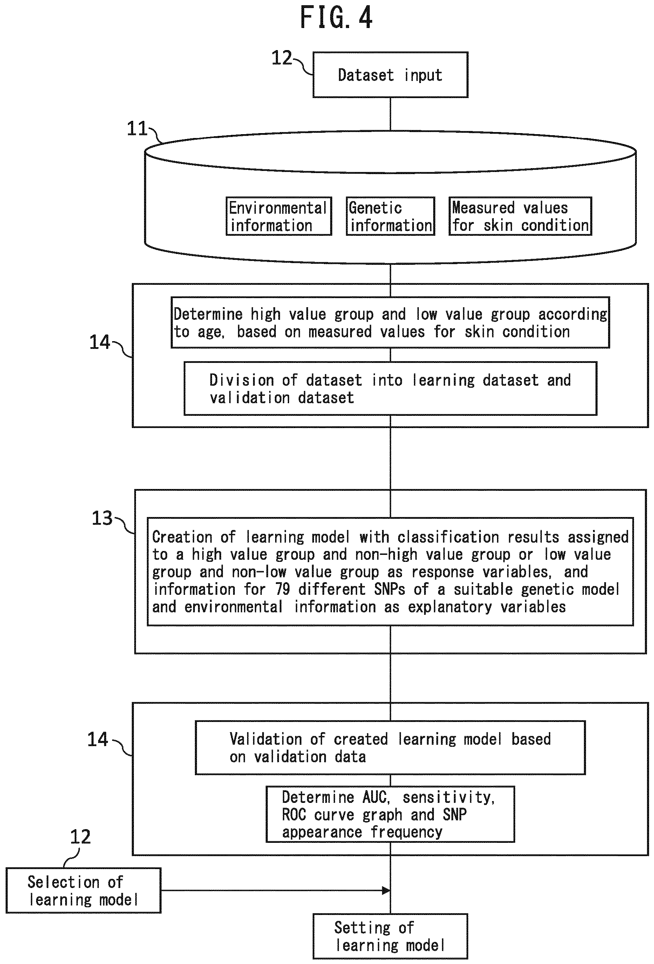

Fig. 2 and Fig. 4 are schematic diagrams showing an example of the flow of processing carried out at the input unit 12, storage unit 11, processing unit 14 and learning unit 13, for learning model creation. A dataset including genetic information and environmental information, and measured values for a skin condition which have been measured in advance, is inputted through the input unit 12, and is processed at the processing unit 14 and learning unit 13 after having been stored in the storage unit 11. At the processing unit 14, the dataset is divided into a learning dataset and a validation dataset, but according to another aspect a separate validation dataset may be provided. At the learning unit 13, inputted measured values for a skin condition or preprocessed measured value data are used as response variables, while the environmental information and genetic information are used as explanatory variables, to create a learning model which is weighted to obtain response variables. Variables may also be selected before creation of the learning model.

SNPs are generally classified as recessive homozygous, heterozygous or dominant homozygous, based on genotype, but one SNP may have interaction due to the existence of another SNP. Each SNP is therefore analyzed with 4 genetic models: recessive model, dominant model, additive model or multiplicative model, using the explanatory variables, allowing selection of the optimal model for each SNP. The analysis can be carried out by regularization using Elastic Net. Determining a genetic model before creating a learning model according to the invention is expected to increase precision for determining skin type based on SNPs.

According to one embodiment, explanatory variables are weighted with the measured values for a skin condition as response variables, and with the respective explanatory variables being the 79 types of SNP information and at least one item of environmental factor information selected from the group consisting of age, body height, body weight, BMI, ultraviolet exposure information and smoking information (Fig. 2). The weighting may be done by multiple regression analysis. When individual genetic information and environmental information are inputted to the learning unit which includes the learning model created in this manner, the learning unit can output a skin condition score. The learning unit that outputs a skin condition score may therefore be referred to as a learning unit that determines the skin condition score. By inputting environmental information with varied age, the learning unit also outputs a future skin condition score. By inputting genetic information and age-varied environmental information into the learning unit 13, the processing unit can thus create a graph representing change in skin condition with age as a variable, based on the skin condition score outputted from the learning unit 13. Further, by inputting varied behavioral factors such as smoking or ultraviolet ray countermeasures among environmental information into the learning unit, the learning unit can output a skin condition score reflecting those behavioral factors, allowing future skin condition to be predicted. The learning unit which determines a skin condition score can thus be referred to as a learning model that predicts changes in skin condition.

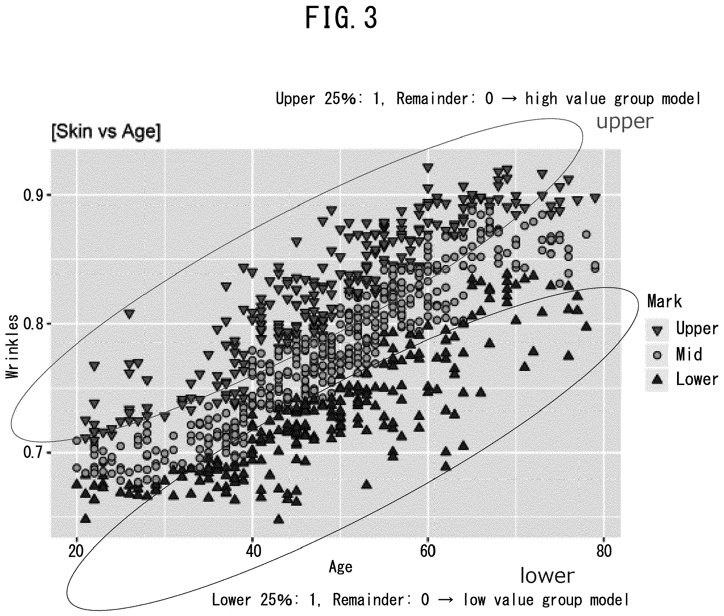

According to another embodiment, preprocessed data for measured values for a skin condition for a test subject can be used as response variables. As an example of preprocessing, based on the measured values for a skin condition, fixed proportions for the high value side or low value side are set as a high value group or low value group, and subjects are classified into a high value group and non-high value group or a low value group and non-low value group according to their skin conditions (Fig. 3). Assignment to the high value group or low value group may be used as a response variable (Fig. 3). Classification into the high value group and non-high value group or low value group and non-low value group will usually be done by the processing unit 14. The high value group and low value group may be set according to age, with age widths of 10 years, 5 years and 2 years, for example. The high value group and low value group may be an X% high value group and X% low value group, as one example, where X% may be selected as any numerical value, or may be selected from the group consisting of 5%, 10%, 15%, 20%, 25% and 30%, for example. Age width and X may also differ depending on the selected skin condition.

After the preprocessing, factors contributing to the high value group, for a high value group and non-high value group, can be determined by the learning unit 13 by logistic analysis from SNP information and/or environmental factor information for a test subject. As an example, a test subject in the high value group for a certain skin condition may be defined as 1 and a test subject in the non-high value group may be defined as 0, as response variables, and a learning model can be created with weighting for explanatory variables from among 79 different SNP information items, and environmental factor information. Similarly, the learning unit 13 can determine factors contributing to the low value group, for a low value group and non-low value group, by logistic analysis from SNP information and/or environmental factor information for a test subject. As an example, test subjects in the low value group for a certain skin condition may be defined as 1 and test subjects in the non-low value group may be defined as 0, as response variables, and a learning model can be created with weighting for explanatory variables from among 79 different SNP information items, and/or environmental factor information. Construction of the learning model can be carried out by logistic regression. For each skin condition, a learning model is constructed for high value group or low value group assessment, with the learning unit 13 retaining the learning models. By inputting SNP information and/or environmental factor information for a test subject to the learning unit 13 containing the learning models, the learning unit 13 outputs assignment of the subject to the high value group or low value group for each skin condition. As one example, if test subjects in the high value group are defined as 1 for a certain skin condition while test subjects in the non-high value group are defined as 0, as response variables, then the possibility that the subject is assigned to the high value group is outputted as a numerical value in the range of 0 or 1. Similarly, if test subjects in the low value group are defined as 1 for a certain skin condition while test subjects in the non-low value group are defined as 0, as response variables, then the possibility that the subject is assigned to the low value group is outputted as a numerical value in the range of 0 or 1. By thus creating a learning model for high value group/low value group assessment for a high value group and low value group of a certain skin condition, it is possible to determine skin type from both the possibility of assignment to the high value group and the possibility of assignment to the low value group for the skin condition.

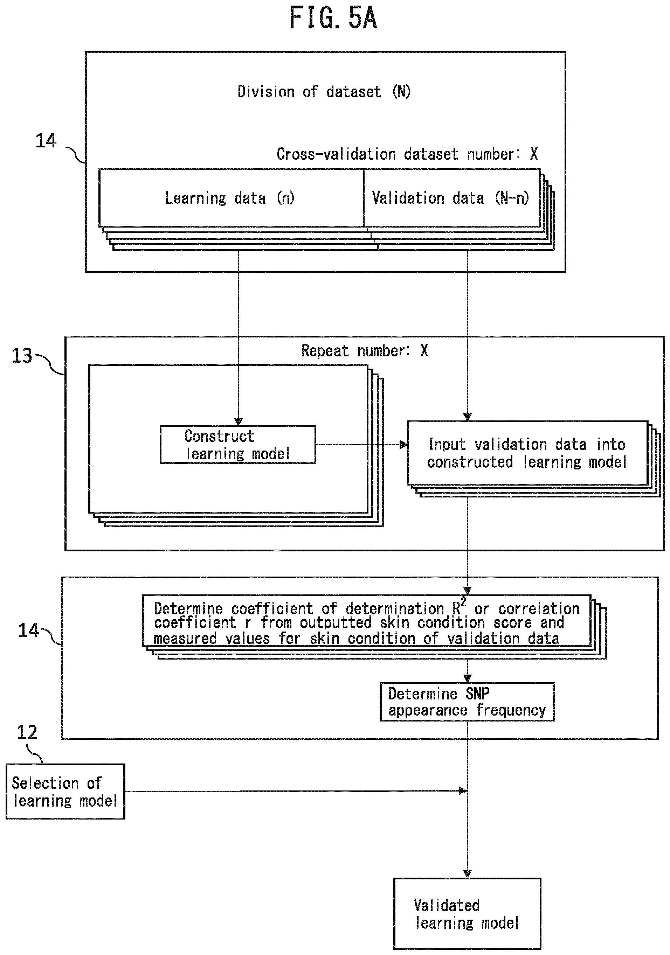

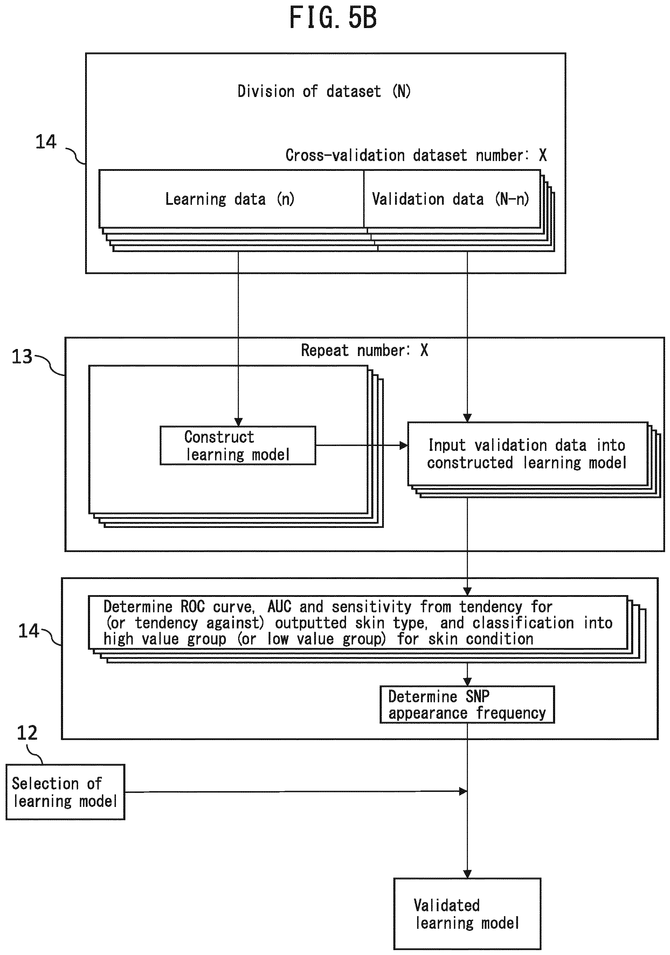

Before learning at the learning unit 13, the dataset that includes SNP information and/or environmental factor information of a test subject, and digitized information for the skin condition of the test subject, may be divided into a learning dataset and validation dataset. By dividing the dataset into a learning dataset and validation dataset, it is possible to construct a cross-validation dataset for an arbitrary number x (Fig. 5). The preprocessing may be carried out by the processing unit 14. The preprocessing allows learning processing/verification processing to be carried out multiple times based on a dataset N, and allows construction of a more highly precise model even for limited datasets.

The method of constructing the learning model that includes preprocessing by dividing into a learning dataset and validation dataset includes the following steps:
(i) a step of selecting a learning dataset n,
(ii) a step of dividing into a learning dataset n and a validation dataset N-n,
(iii) a step of constructing a learning model for the learning dataset n, and
(iv) a step of validating the constructed model using the validation dataset N-n, and it can be carried out by selecting a desired number of times X and a learning dataset (n₁ ... n_{X}), and repeating steps (i) to (iv). The construction method can be carried out with the information processing device 10/system 20.

Specifically, the information processing device/system comprises:
a storage unit that stores a dataset N including explanatory variables and response variables,
a processing unit that selects part of the stored dataset as a learning dataset and inputs it to the learning unit, while inputting explanatory variables of a validation dataset into a learning unit that includes a constructed learning model and validating the learning model based on response variables of the validation dataset of the outputted values, and
a learning unit that constructs a learning model that has been trained to output a response variable for an inputted explanatory variable, from the inputted explanatory variables and response variables of the learning dataset,
   wherein the following steps are included:
   (i) a step in which the processing unit selects a learning dataset n from a dataset N stored in the storage unit, divides it into a learning dataset n and a validation dataset N-n, and inputs the learning dataset n into the learning unit,
   (ii) a step in which the learning unit constructs a learning model trained to output a response variable for an inputted explanatory variable, for the learning dataset n,
   (iii) a step in which the processing unit inputs the validation dataset N-n into the learning unit that includes the constructed learning model, and
   (iv) a step in which the learning unit uses the outputted value and the response variable of the validation dataset N-n to validate the constructed model, and
a step in which a desired number of times X and learning dataset (n₁ ... n_{X}) are selected and steps (i) to (iv) are repeated.

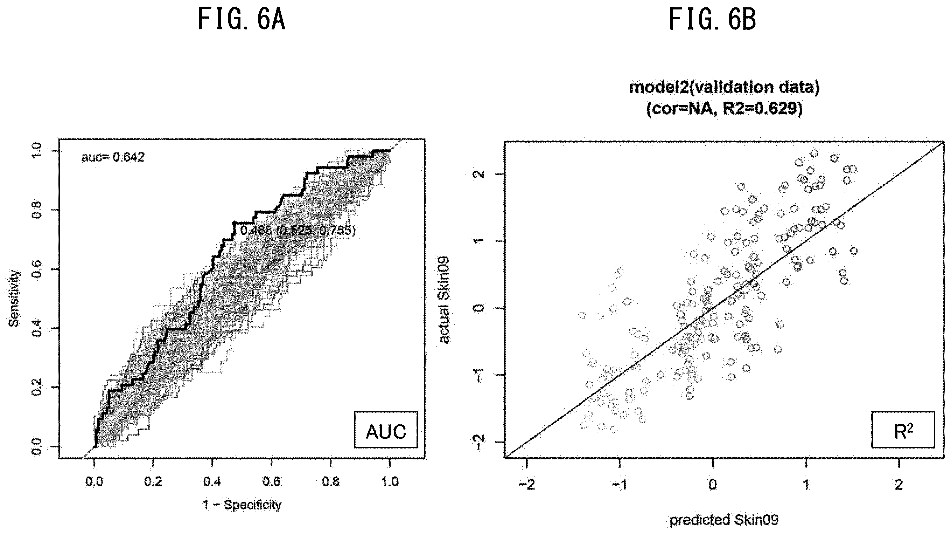

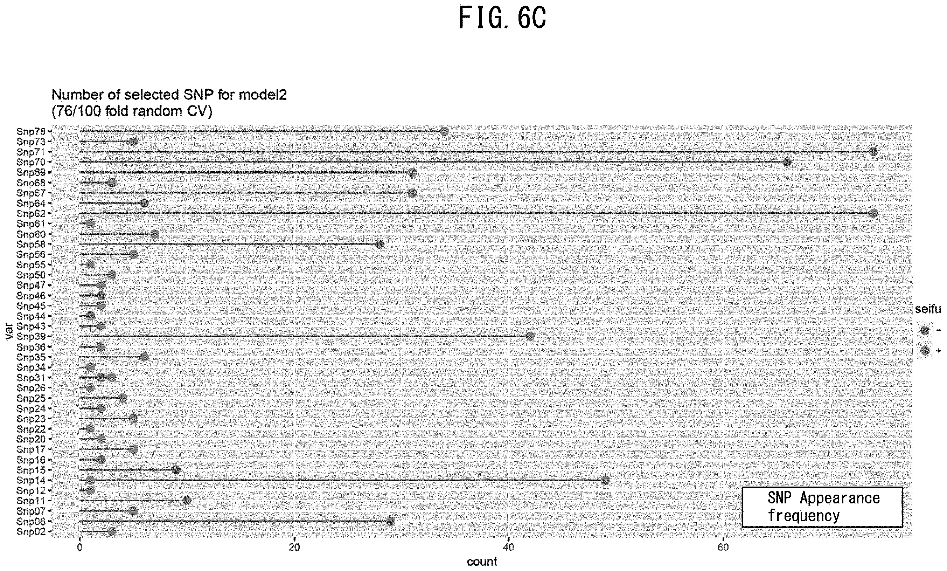

The validation may be made by inputting the validation data into the constructed learning model and performing statistical processing of the outputted value and the response variables of the validation data. The validation may be by drawing a receiver operating characteristics (ROC) curve or calculating a coefficient of determination R2 or correlation coefficient r. Since the dataset is divided and there are x cross-validation datasets, a maximum of x statistical processing events are obtained for a maximum of x learning models created (Figs. 6A and B). An appearance frequency is represented for each SNP for the maximum of x learning models created (Fig. 6C). An optimal learning model can be selected based on this information, and also in consideration of additional biochemical knowledge.

According to one aspect, when the dataset N is divided into the learning dataset n and the validation dataset N-n, it may be distributed so that age, ultraviolet exposure information, smoking information and skin measurement test facilities, among the explanatory variables for multivariate analysis, are as equal as possible. This will allow a more highly precise model to be constructed. When the number of data items under different conditions is not at least 2, then the data is distributed as learning data. This will help to solve problems of disproportion of the learning dataset and validation dataset in the step of constructing a model based on multivariate analysis and the step of validating the constructed model (problems such as inability to estimate the coefficient of regression of multivariate analysis which prevents model construction, or overfitting to the learning dataset which results in insufficient generalizability of the model).

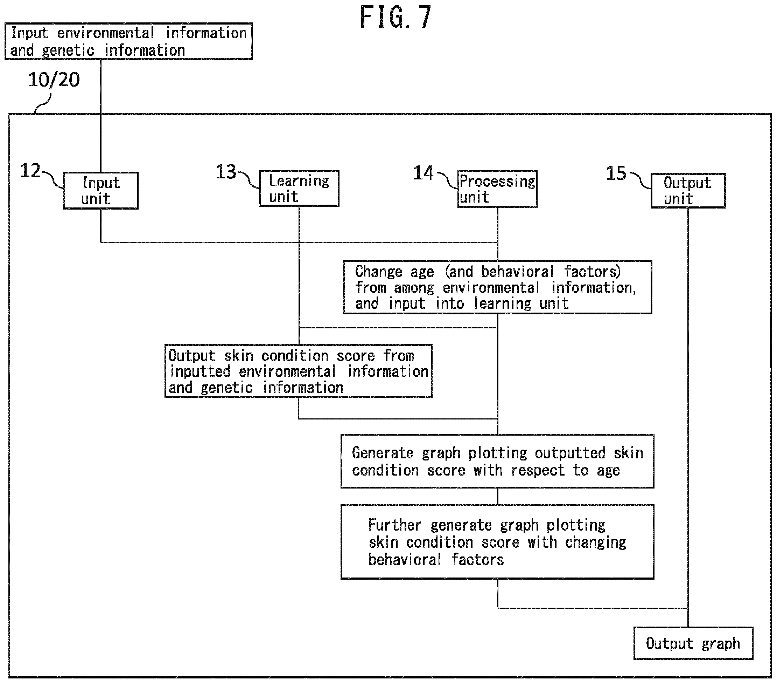

The flow of input of genetic information and environmental information by the evaluation system of the invention to predict future skin condition is shown in Fig. 7. The method, evaluation system and program of the invention utilize a constructed learning model that predicts changes in skin condition. The method, evaluation system and program of the invention also allow input of environmental information and genetic information through the input unit 12. The inputted environmental information and genetic information are sent to the learning unit 13 and a skin condition score is outputted by the learning unit 13. At the processing unit, the environmental information of age and behavioral factors are changed and inputted into the learning unit, causing the learning unit to output a skin condition score for each case. Plotting of the skin condition score outputted for an age at the processing unit 14 allows generation of a graph for change in skin condition score according to age, or generation of a graph for change in skin condition score according to a behavioral factor. The graph generated at the processing unit 14 is sent to the output unit 15 and a graph is outputted at the output unit 15. The results for processing in each step may also be stored in the storage unit 11.

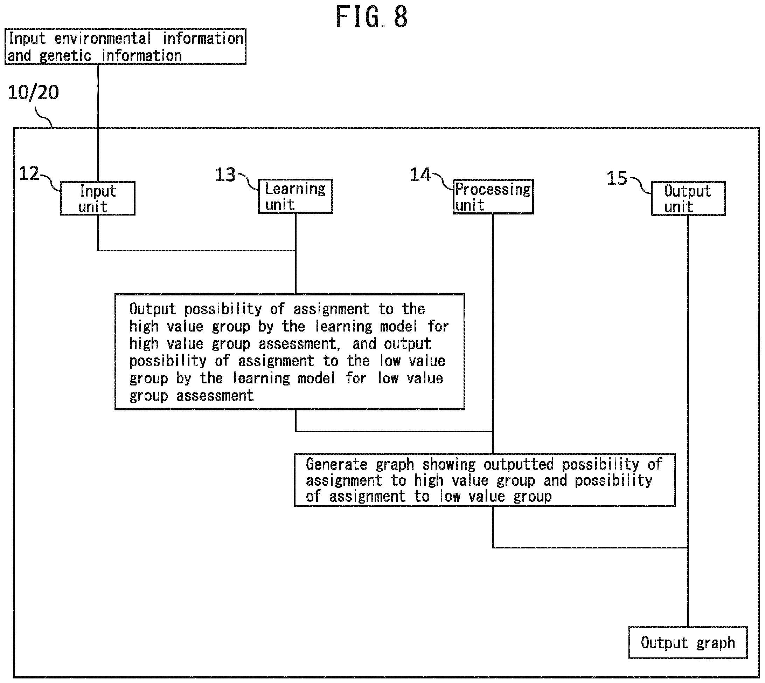

The flow of input of genetic information and environmental information by the evaluation system of the invention to assess skin type is shown in Fig. 8. The method, evaluation system and program of the invention utilize a constructed learning unit that assesses skin type. The learning unit that assesses skin type has a learning model for high value group assessment that outputs the possibility of assignment to the high value group, and a learning model for low value group assessment that outputs the possibility of assignment to the low value group. The method, evaluation system and program of the invention also allow input of environmental information and genetic information through the input unit 12. The inputted environmental information and genetic information are sent to the learning unit 13, and the learning unit 13 outputs the possibility of assignment to the high value group by the learning model for high value group assessment of the learning unit 13, and also outputs the possibility of assignment to the low value group by the learning model for low value group assessment. The processing unit 14 draws a graph showing a possibility of assignment to the high value group and possibility of assignment to the low value group which have been outputted. The graph generated at the processing unit 14 is sent to the output unit and a graph is outputted at the output unit 15. The results for processing in each step may also be stored in the storage unit 11.

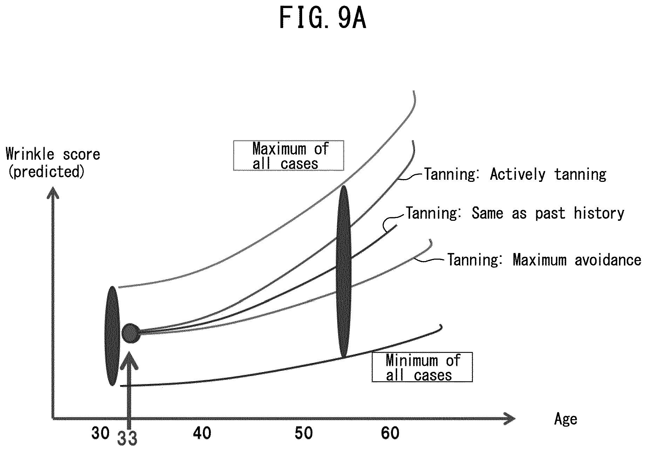

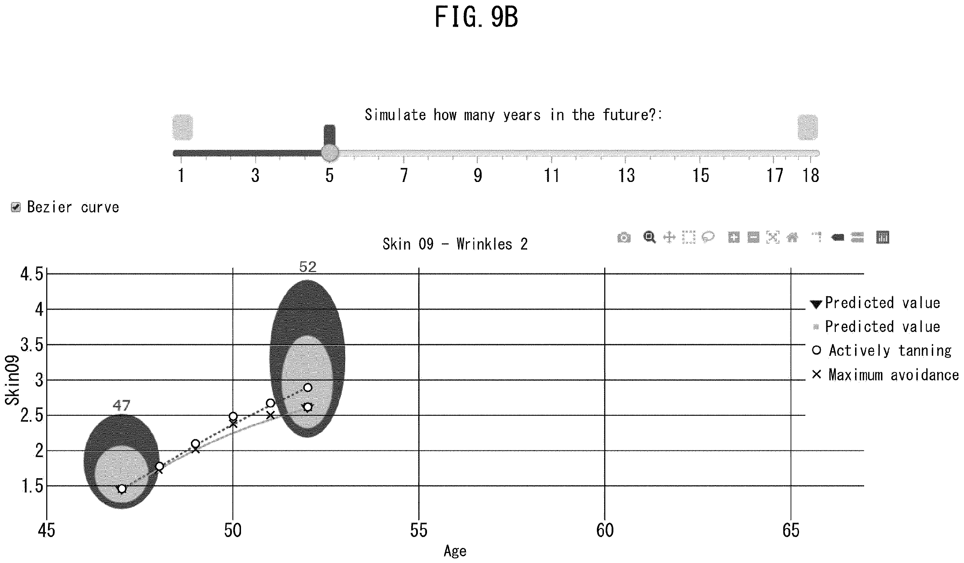

When a learning model that predicts change in skin condition is used, the formula for outputting the skin condition score at the learning unit 13 may also be outputted. Using the formula, a skin condition change prediction graph can be generated at the processing unit 14, representing age on the X-axis and skin condition score on the Y-axis. It is also possible to generate a skin condition change prediction graph for altered behavioral factors, such as smoking habit or ultraviolet ray countermeasures (Fig. 9). Fig. 9A is a change prediction graph for a skin condition (wrinkles) with variable age, where "wrinkles" has been selected as an example of a skin condition, and it also shows change due to tanning countermeasures. Fig. 9B is an example of a display to be shown to a customer, where a future date (years later) is selected to allow change in skin condition (wrinkle) score to be visually displayed. Change in the future skin condition score can also be displayed by inputting a behavioral factor such as ultraviolet ray countermeasures or smoking information. These graphs may also be displayed on a shop terminal, or on the internet through a shop terminal. The analysis results may also be printed on paper as a report and sent by mail. In regard to ultraviolet ray countermeasures, such as the use of sunscreen as a tanning countermeasure, for example, the change in skin condition can be displayed for: maximum avoidance, continuance of past usage, or actively tanning.

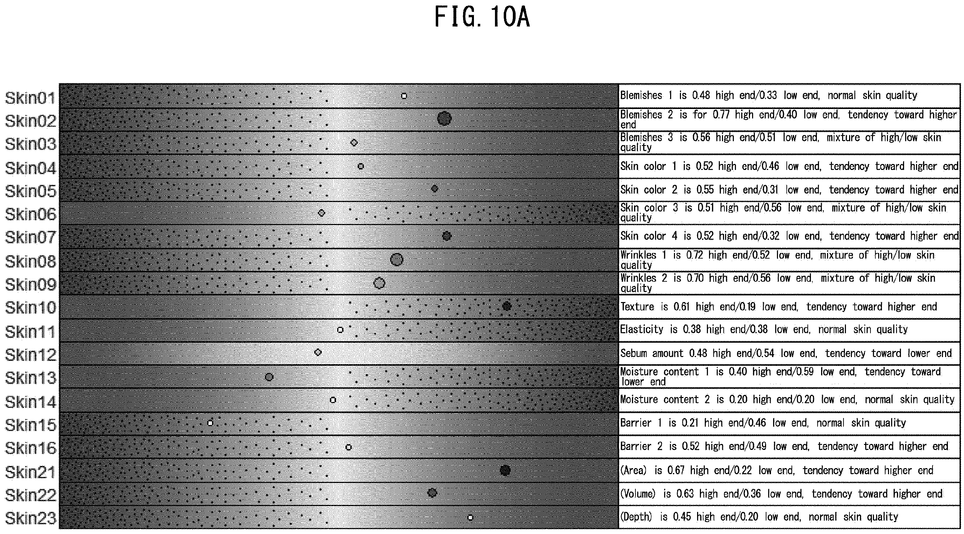

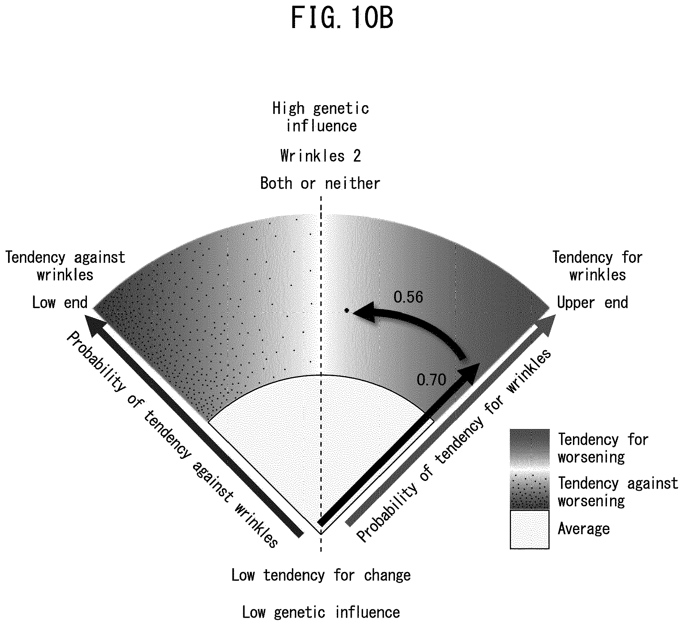

When the learning unit 13 that assesses skin type has been used, the possibility of assignment to the high value group and/or low value group for skin type may be outputted by the learning unit 13. As an example, when "pigmented spot" has been selected as the skin condition, the high value group of measured pigmented spot values (pigmented spot index according to Visia) is an indicator of tendency for pigmented spots, while the low value group of measured pigmented spot values (pigmented spot index according to Visia) is an indicator of tendency against pigmented spots. When environmental information and genetic information are inputted into the learning unit 13, therefore, it is possible to assess skin type in terms of pigmented spots from the viewpoint of both tendency for (possibility of assignment to the high value group) and tendency against (possibility of assignment to the low value group) pigmented spots. The processing unit 14 may process the possibility of assignment to the high value group and possibility of assignment to the low value group as determined by the learning unit 13, and may generate graphs representing tendency for (possibility of assignment to the high value group) and tendency against (possibility of assignment to the low value group) (Fig. 10A), or may generate a sector graph with tendency for (possibility of assignment to the high value group) and tendency against (possibility of assignment to the low value group) on perpendicular axes (Fig. 10B).

Counseling may be provided based on the skin type or future skin condition outputted from the output unit 15. For counseling, a beauty component or its concentration suited for the skin type, or a cosmetic suited for the skin type, may be displayed. Information regarding usage frequency may also be provided in addition to the cosmetic suited for the skin type. The relationship between skin type, beauty components and their concentrations or cosmetics may be stored in the storage unit 11 beforehand as a table, or it may be combined with skin type output and provided as information for beauty components and their concentrations, or cosmetics.

Environmental factor information is inputted as the results of a questionnaire survey. A questionnaire may be at least one type of information selected from the group consisting of age, body height, body weight, ultraviolet exposure information and smoking information, for example. Ultraviolet exposure history information and smoking information are obtained by the questionnaire. Ultraviolet exposure information is obtained by questions regarding current tanning, and by questions regarding past tanning. As an example, classification may be on 4 levels in a questionnaire regarding current tanning, or on 3 levels for a questionnaire regarding past tanning. For smoking information, current or past smoking behavior, and in case of smoking, the daily frequency and duration of smoking, may be obtained. As an example, the survey may be on 3 levels: no history of smoking, past history of smoking 20 or more cigarettes per day, and past history of smoking less than 20 cigarettes per day. Information regarding current tanning among ultraviolet exposure information, and current smoking information, may be used as behavioral factors to predict changes in future skin condition.

Measured values for a skin condition are measured by selecting appropriate measuring means for the skin condition being evaluated. Specifically, measured values for a skin condition selected from the group consisting of pigmented spots, wrinkles, skin firmness, moisture, texture and tone, and sebum are used. Such skin conditions can also be classified into subgroups. For example, pigmented spots can be classified into measured values according to Visia (Visia pigmented spot index), and number of pigmented spots and classification of pigmented spot area, based on a skin imaging device. Wrinkles and firmness can be classified into measured wrinkle values according to Visia (Visia wrinkle index), replica analysis (eye corner wrinkles) and skin elasticity values. Moisture content can be classified into stratum corneum moisture content and transepidermal water loss (barrier function), using a specialized commercial device. Skin color can be classified into cheek color (melanin, skin brightness, yellowness) and skin color within the inner upper arm, determined using a spectrophotometer. The measured values are obtained using any means known in the technical field, suited for the skin condition to be measured. As one example, pigmented spots, wrinkles and texture can be measured as digitized values, using a skin imaging device such as VISIA^{R}. Skin moisture and sebum can be measured as digitized values using a skin meter such as a Corneometer, Vapometer or Sebumeter.

Skin condition can be determined based on at least one property selected from the group consisting of pigmented spots, wrinkles, firmness, skin moisture, texture, skin color and sebum. A SNP may be selected according to the skin condition to be determined. SNP information to be used for the invention consists of the following 79 types of SNP information: rs1800629, rs2108622, rs1047781, rs12203592, rs16891982, rs3760776, rs9340799, rs12913832, rs17822931, rs964184, rs1801133, rs2228479, rs10515552, rs10741657, rs10882272, rs11057830, rs11234027, rs12272004, rs12377462, rs12785878, rs12931267, rs1540771, rs1667255, rs1993116, rs2060793, rs2227564, rs2282679, rs2298585, rs3829251, rs41281112, rs4654748, rs492602, rs602662, rs1030868, rs1126643, rs1256062, rs12934922, rs1485766, rs1501299, rs17577, rs1799724, rs18,00012, rs1800414, rs2010963, rs2234693, rs2241145, rs2285053, rs2287074, rs2287076, rs2987983, rs3918242, rs4065, rs4252125, rs6152, rs7201, rs74653330, rs7501331, rs8110862, rs8326, rs833061, rs1050565, rs1799750, rs4880, rs1050450, rs6058017, rs1061622, rs2046571, rs2232228, rs3785079, rs2246416, rs1107946, rs11568737, rs41303970, rs12051272, rs182052, rs3865188, rs6810075, rs7799039, rs1137101.

As an example, when the skin condition is pigmented spots, learning models may be constructed for the measured pigmented spot value according to Visia (Visia wrinkle index), the number of pigmented spots determined by a skin image analyzer, and pigmented spot area. SNPs used for evaluation of these skin conditions are shown below. One or more SNPs selected from the group consisting of the SNPs listed in the following tables can be used for learning model creation and for evaluation of skin condition (pigmented spots) using the learning model.

### [Table 1]

**Table 1: Pigmented spots 1 (Visia pigmented spot index)**

| | |
|---|---|
| **Skin01_linear_m1m2** | **rs1030868, rs1050450, rs10741657, rs10882272, rs1107946, rs1126643, rs12051272, rs1256062, rs12785878, rs1501299, rs1540771, rs1799724, rs1799750, rs1800414, rs1801133, rs 2010963, rs2046571, rs2060793, rs2108622, rs2228479, rs22 34693, rs2246416, rs2282679, rs2285053, rs2287074. rs2287 076, rs2987983, rs3760776, rs3785079, rs4065, rs4654748, rs6058017, rs7201, rs74653330, rs8110862, rs8326, rs83306 1, rs964184** |
| **Skin01_upper_m1m2** | **rs1030868, rs1050450, rs10515552, rs10741657, rs10882272, rs11057830, rs1107946, rs12377462. rs1256062, rs129349 22, rs1485766, rs1501299, rs1540771, rs1800414, rs199311 6, rs2010963, rs2046571, rs2060793, rs2108622, rs2227564 rs2234693, rs2241145, rs2246416, rs2285053, rs2287074 rs2287076, rs2987983, rs3785079, rs3829251, rs3865188 , rs4654748, rs7201, rs74653330, rs7501331, rs8110862, r s8326, rs833061, rs964184** |
| **Skin01_lower_m1m2** | **rs1047781, rs1050450, rs10741657, rs1107946, rs1137101, rs12051272, rs1256062, rs12934922, rs1485766, rs1667255 , rs1799724, rs1799750, rs1801133, rs182052, rs2010963, rs2046571, rs2108622, rs2234693, rs2246416, rs2285053, r s2287074, rs2287076, rs2987983, rs3760776, rs3865188, rs 6810075, rs7201, rs74653330, rs7799039, rs8110862, rs833 061, rs9340799, rs964184** |

### [Table 2]

**Table 2: Pigmented spots 2 (Pigmented spot count)**

| | |
|---|---|
| **Skin02_linear_m1m2** | **rs1030868, rs1061622, rs10741657, rs11057830, rs1107946 , rs1126643, rs12051272, rs12377462. rs1256062, rs148576 6, rs1501299. rs1540771, rs1799750, rs1800414, rs1801133 , rs182052, rs2046571, rs2060793, rs2108622, rs2234693, rs2282679, rs2285053, rs2287074, rs2287076, rs2298585, r s2987983, rs3785079, rs3865188, rs4065, rs4880, rs681007 5, rs7201, rs74653330, rs7799039, rs8110862, rs833061** |
| **Skin02_upper_m1m2** | **rs10741657, rs1107946, rs1137101, rs12051272, rs1256062 , rs1485766, rs1667255, rs1799724, rs1799750, rs182052, rs2046571, rs2060793, rs2234693, rs2246416, rs2282679, r s2287074, rs2287076, rs2987983, rs3785079, rs3865188, rs 41303970, rs6810075, rs7201, rs833061, rs9340799** |
| **Skin02_lower_m1m2** | **rs1030868, rs1047781, rs1050450, rs1061622, rs10741657, rs11057830, rs1107946, rs11234027, rs12377462, rs1256062 , rs12785878, rs1540771, rs1667255, rs1799750, rs1801133 , rs182052, rs2010963, rs2046571, rs2060793, rs2108622, rs2282679, rs2285053, rs2987983, rs4065, rs4880, rs68100 75, rs7201, rs8110862, rs8326, rs833061, rs9340799** |

### [Table 3]

**Table 3: Pigmented spots 3 (Pigmented spot area)**

| | |
|---|---|
| **Skin03_linear_m1m2** | **rs1061622, rs11057830, rs1107946, rs11234027, rs1126643 , rs1137101, rs12051272, rs12377462, rs1256062, rs127858 78, rs12934922, rs1485766, rs1540771, rs1667255, rs17822 931, rs1799750, rs1800414, rs2046571, rs2108622, rs22275 64, rs2234693, rs2241145, rs2246416, rs2282679, rs229858 5, rs3785079, rs3829251, rs3865188, rs4880, rs6058017, r s6810075, rs74653330, rs8110862, rs8326** |
| **Skin03_upper_m1m2** | **rs1030868, rs1047781, rs1061622, rs10882272, rs11234027, rs1137101, rs12377462. rs12785878, rs12934922, rs1501299, rs1540771, rs17577, rs1799750, rs1800414, rs2010963, rs20 46571, rs2227564, rs2234693, rs2241145, rs2246416, rs2282 679, rs2285053, rs2287074, rs2287076, rs2987983, rs376077 6, rs3785079, rs3829251, rs4654748, rs6058017, rs7201, rs 833061, rs9340799, rs964184** |
| **Skin03_lower_m1m2** | **rs1047781, rs1050565, rs1061622, rs10741657, rs1107946, r s11234027, rs1126643, rs1137101, rs12051272, rs12785878, rs1485766, rs1667255, rs1801133, rs2046571, rs2108622, rs 2227564, rs2228479, rs2234693, rs2241145, rs2246416, rs22 82679, rs2285053, rs2287074, rs2987983, rs3785079, rs3865 188, rs41281112, rs4880, rs6058017, rs74653330, rs8110862 , rs8326, rs833061, rs964184** |

As another example, when the skin condition is skin color, learning models may be created for melanin content, skin brightness and yellowness, as measured using a spectrophotometer. SNPs used for evaluation of these skin conditions are shown below. One or more SNPs selected from the group consisting of the SNPs listed in the following tables can be used for learning model creation and for evaluation of skin condition (skin colors 1 to 4) using the learning model.

### [Table 4]

**Table 4: Skin color 1 (melanin amount, cheek)**

| | |
|---|---|
| **Skin04_linear_m1m2** | **rs1030868, rs1047781, rs10741657, rs1107946, rs1126643, rs1137101, rs12377462, rs1256062, rs12785878, rs1485766 , rs1540771, rs1799750, rs1800414, rs1993116, rs2010963 , rs2060793, rs2108622, rs2227564, rs2232228, rs2234693 , rs2241145, rs2246416, rs2282679, rs2285053, rs2287074 , rs2287076, rs2987983, rs3760776, rs3785079, rs3829251 , rs4065, rs4654748, rs4880, rs6058017, rs7201, rs746533 30, rs7501331, rs964184** |
| **Skin04_upper_m1m2** | **rs1030868, rs1047781, rs10741657, rs10882272, rs1107946 , rs12051272, rs12377462, rs12785878, rs12934922, rs1485 766, rs1540771, rs1799750, rs1800414, rs1993116, rs20109 63, rs2046571, rs2108622, rs2227564, rs2228479, rs223222 8, rs2234693, rs2241145, rs2246416, rs2282679, rs2287074 , rs2287076, rs2298585, rs3760776, rs3785079, rs3865188 , rs4880, rs6058017, rs7201, rs74653330, rs8110862, rs96 4184** |
| **Skin04_lower_m1m2** | **rs1047781, rs1050450, rs1050565, rs10515552, rs10741657 , rs1107946, rs1126643, rs12377462, rs1256062, rs1278587 8, rs1501299, rs1540771, rs17577, rs1799724, rs1800414, rs1801133, rs1993116, rs2010963, rs2046571, rs2060793, r s2108622, rs2227564, rs2232228, rs2234693, rs2241145, rs 2246416, rs2287074, rs2287076, rs3760776, rs3785079, rs3 829251, rs4065, rs41281112, rs41303970, rs4880, rs605801 7, rs7201, rs74653330, rs8326, rs9340799** |

### [Table 5]

**Table 5: Skin color 2 melanin amount inner upper arm)**

| | |
|---|---|
| **Skin05_linear_m1m2** | **rs74653330, rs1800414, rs1540771, rs1126643, rs2060793, rs1047781, rs2987983, rs7501331, rs2010963, rs1993116, r s11234027, rs6058017, rs9340799, rs2287074, rs2246416, r s3829251, rs10741657, rs3785079, rs2234693, rs11057830, rs182052, rs1799750, rs6810075, rs12051272, rs1501299, r s1485766, rs2285053, rs2282679, rs12934922, rs8110862, r s10882272** |
| **Skin05_upper_m1m2** | **rs74653330, rs7501331, rs2010963, rs1540771, rs1800414, rs9340799, rs2234693, rs182052, rs3829251, rs6810075, rs 2246416, rs12785878, rs10741657, rs2241145, rs1126643, r s6058017, rs2285053, rs3785079, rs964184, rs833061, rs12 56062, rs1799750, rs1047781, rs2046571, rs1501299, rs206 0793, rs11451830, rs2282679, rs1137101, rs7201, rs4880, rs11234027, rs1485766, rs12051272, rs1993116, rs1801133 , rs2108622** |
| **Skin05_lower_m1m2** | **rs74653330, rs2234693, rs9340799, rs1800414, rs1540771, rs1126643, rs3760776, rs8110862, rs2232228, rs2282679, r s964184, rs2228479, rs10741657, rs2060793, rs3785079, rs 7501331, rs1501299, rs2298585, rs2987983, rs2241145, rs1 2934922, rs1047781, rs1107946, rs2010963, rs1061622, rs1 2377462, rs2046571, rs1050565, rs1799724, rs1256062, rs1 993116, rs2285053, rs182052, rs6058017, rs1801133, rs832 6, rs1799750, rs2246416, rs4065, rs3829251, rs1667255, r s1137101** |

### [Table 6]

**Table 6: Skin color 3 (skin brightness)**

| | |
|---|---|
| **Skin06_linear_m1m2** | **rs74653330, rs1800414, rs3785079, rs12785878, rs2234693 , rs1126643, rs2241145, rs6058017, rs1137101, rs1030868 , rs1501299, rs1047781, rs2246416, rs1540771, rs17577, r s1667255, rs3760776, rs6810075, rs1256062, rs7201, rs750 1331, rs2287074, rs182052, rs8326, rs1107946, rs12051272 , rs1801133, rs41281112, rs2108622** |
| **Skin06_upper_m1m2** | **rs74653330, rs1800414, rs6810075, rs8326, rs1256062, rs1 2785878, rs2285053, rs2282679, rs3760776, rs41281112, rs 1540771, rs3785079, rs2234693, rs2046571, rs2241145, rs2 227564, rs1137101, rs2987983, rs833061, rs1030868, rs150 1299, rs1107946, rs7201, rs1667255, rs964184, rs4065, rs 17577, rs3829251, rs1126643, rs10882272, rs1485766, rs22 98585, rs182052, rs1799724, rs10741657, rs2246416, rs123 77462, rs3865188, rs2232228, rs1047781, rs6058017, rs120 51272, rs1050450, rs10515552** |
| **Skin06_lower_m1m2** | **rs1800414. rs74653330, rs6058017, rs12785878, rs2246416 , rs1137101, rs17577, rs2234693, rs1485766, rs1061622, r s3785079, rs10515552, rs3829251, rs2228479, rs41303970, rs1047781, rs2241145, rs7501331, rs2285053, rs4654748, r s2287076, rs1030868, rs1126643, rs11234027, rs1256062, r s2108622, rs2232228, rs6810075, rs1540771, rs833061, rs4 880, rs4065** |

### [Table 7]

**Table 7: Skin color 4 (yellowness)**

| | |
|---|---|
| **Skin07_linear_m1m2** | **rs2287074, rs74653330, rs1800414, rs11057830, rs2060793 rs8326, rs12377462, rs2010963, rs2282679, rs6058017, r s1485766, rs1047781, rs1126643, rs6810075, rs10515552, r s2108622, rs11234027, rs10741657, rs2287076, rs2285053, rs3785079, rs2234693, rs1540771, rs4654748, rs2241145, r s1050565, rs182052, rs1799750, rs9340799, rs4880, rs1256 062, rs1993116, rs3829251, rs1667255, rs2227564, rs22322 28** |
| **Skin07_upper_m1m2** | **rs2287076, rs11057830, rs2287074, rs74653330, rs2010963 , rs6058017, rs2234693, rs2241145, rs2282679, rs1256062 , rs2060793, rs12377462. rs11234027, rs833061, rs1485766 , rs2108622, rs4654748, rs2228479, rs8110862, rs10515552 , rs1501299, rs1030868, rs10741657, rs7201, rs1800414, r s3760776, rs1107946, rs8326, rs1126643, rs182052, rs7799 039, rs6810075, rs3785079, rs1799750, rs2285053, rs96418 4, rs1667255, rs4065, rs41281112, rs2227564, rs1801133, rs2232228** |
| **Skin07J_lower_m1m2** | **rs8326, rs2287074, rs74653330, rs1256062, rs1047781, rs1 799750, rs3785079, rs1800414, rs182052, rs2285053, rs228 2679, rs6810075, rs12051272, rs2108622, rs1485766, rs222 8479, rs1050565, rs2232228, rs2010963, rs12377462, rs222 7564, rs1126643, rs2060793, rs1667255, rs10515552, rs105 0450, rs10741657, rs1540771, rs2287076, rs7799039, rs720 1, rs17822931, rs3865188** |

As another example, when the skin condition is wrinkles, measured wrinkle values according to Visia (Visia wrinkle index) may be used to construct two different parent populations, and learning models may be constructed for each. SNPs used for evaluation of these skin conditions are shown below. One or more SNPs selected from the group consisting of the SNPs listed in the following tables can be used for learning model creation and for evaluation of skin condition (wrinkles 1 and 2) using the learning model.

### [Table 8]

**Table 8: Wrinkles 1 (Visia wrinkle index)**

| | |
|---|---|
| **Skin08_linear_m1m2** | **rs7799039, rs2232228, rs12377462. rs1256062, rs3760776, rs6810075, rs1061622, rs2234693, rs1107946, rs12785878, rs2228479, rs6058017, rs2246416, rs1667255, rs1799724, r s1030868, rs182052, rs4654748, rs1126643, rs1799750, rs1 501299, rs2108622, rs11234027, rs2287076, rs17822931, rs 2298585, rs8326, rs1485766, rs2987983, rs11057830, rs488 0, rs1801133, rs7201, rs2227564, rs10741657, rs41281112 , rs964184, rs7501331, rs2282679, rs12051272, rs3865188 , rs10515552, rs833061, rs1540771, rs1800414, rs41303970 , rs1137101, rs2046571, rs9340799, rs1047781** |
| **Skin08_upper_m1m2** | **rs1107946, rs7799039, rs10741657, rs1501299, rs1126643, rs2232228, rs3760776, rs12051272, rs2046571, rs1799750, rs8110862, rs12377462. rs2282679, rs1801133, rs11234027 , rs2227564, rs1061622, rs2246416, rs1993116, rs41303970 , rs2987983, rs11057830, rs833061, rs2228479, rs3829251 , rs2060793, rs4654748, rs2298585, rs1030868, rs2287076 , rs1667255, rs1800414, rs1540771, rs2285053, rs1050450 , rs964184, rs2234693, rs2241145, rs1799724, rs7201, rs1 047781, rs2287074** |
| **Skin08_lower_m1m2** | **rs964184, rs2232228, rs1107946, rs1256062, rs7799039, rs 1799724, rs833061, rs12377462. rs6058017, rs2987983, rs7 201, rs2046571, rs182052, rs6810075, rs4654748, rs127858 78, rs2010963, rs41281112, rs2298585, rs2234693, rs11057 830, rs1137101, rs3865188, rs17822931, rs2285053, rs1667 255, rs3785079, rs8326, rs1799750, rs1540771, rs2246416 , rs1061622, rs1047781, rs2287076, rs2227564, rs1030868 , rs1126643, rs3829251, rs8110862, rs1800414, rs1501299** |

### [Table 9]

**Table 9: Wrinkles 2 (Visia wrinkle index)**

| | |
|---|---|
| **Skin09_linear_m1m2** | **rs2108622, rs1256062, rs7799039, rs3760776, rs1799750, rs 6058017, rs12785878, rs2246416, rs6810075, rs2287076, rs1 107946, rs2228479, rs1540771, rs182052, rs11057830, rs123 77462, rs2232228, rs1799724, rs1667255, rs17822931, rs148 5766, rs1501299, rs1126643, rs1801133, rs4654748, rs18004 14, rs2987983, rs4880, rs3865188, rs2227564, rs7501331, r s8326, rs9340799, rs1030868, rs3829251, rs2046571, rs2298 585, rs10515552, rs11234027, rs8110862, rs1050565, rs1061 622, rs964184, rs10741657, rs7201, rs41281112, rs12934922 , rs1993116, rs3785079, rs1047781, rs17577, rs2282679** |
| **Skin09_upper_m1m2** | **rs1799750, rs2246416, rs3785079, rs1107946, rs10741657, r s1501299, rs1993116, rs7799039, rs1801133, rs3760776, rs2 046571, rs11234027, rs1667255, rs8110862, rs10882272, rs8 33061, rs2232228, rs9340799, rs2108622, rs4654748, rs2227 564, rs1126643, rs41303970, rs1050450, rs1030868, rs18004 14, rs74653330, rs2060793, rs2234693, rs2228479, rs298798 3, rs1256062, rs12785878, rs2285053, rs1050565, rs7201, r s11057830, rs1540771, rs2241145, rs2010963** |
| **Skin09_lower_m1m2** | **rs3865188, rs1799724, rs7799039, rs964184, rs12785878, rs 7201, rs3829251, rs6058017, rs1256062, rs2232228, rs12051 272, rs12377462, rs2234693, rs1126643, rs2298585, rs22464 16, rs2287076, rs2046571, rs1107946, rs1800414, rs1485766 , rs41281112, rs1047781, rs8326, rs17822931, rs11057830, rs2987983, rs833061, rs1540771, rs6810075, rs2227564, rs3 785079, rs2010963, rs182052, rs1799750** |

As another example, when the skin condition is texture, learning models may be constructed with measured texture values according to Skinvisiom (Skinvisiom texture score). SNPs used for evaluation of these skin conditions are shown below. One or more SNPs selected from the group consisting of the SNPs listed in the following tables can be used for learning model creation and for evaluation of skin condition (texture) using the learning model.

### [Table 10]

**Table 10: Texture**

| | |
|---|---|
| **Skin10_linear_m1m2** | **rs3785079, rs12377462, rs1256062, rs2232228, rs1107946, rs11057830, rs41303970, rs12785878, rs1485766, rs2241145 , rs3829251, rs2227564, rs8326, rs2282679, rs833061, rs1 1234027, rs1047781, rs4654748, rs6058017, rs8110862, rs1 540771, rs2010963, rs3865188, rs2246416, rs2298585, rs16 67255, r81801133, rs4065, rs7799039, rs1799724, rs103086 8, rs12051272, rs2228479, rs2287074, rs41281112, rs10741 657, rs1993116, rs2285053, rs2108622, rs2060793, rs37607 76** |
| **Skin10_upper_m1m2** | **rs1107946, rs1137101, rs4065, rs2241145, rs1256062, rs12 934922, rs1667255, rs8326, rs964184, rs3785079, rs412811 12, rs2227564, rs2287074, rs2282679, rs2228479, rs382925 1, rs2232228, rs41303970, rs2046571, rs4654748, rs746533 30, rs7201, rs10515552, rs8110862, rs1801133, rs2285053 , rs2287076, rs2234693, rs10741657, rs2060793, rs1501299 , rsl540771, rs7501331, rs12785878, rs11234027, rs179975 0, rs11057830, rs3865188, rs1030868, rs2108622** |
| **Skin10_lower_m1m2** | **rs2298585, rs964184, rs3785079, rs1126643, rs12785878, r s1047781, rs833061, rs1667255, rs3760776, rs1256062, rs4 654748, rs12377462, rs2287076, rs2060793, rs3829251, rs1 7822931, rs2108622, rs2987983, rs10741657, rs8110862, rs 41303970, rs2010963, rs2246416, rs11234027, rs1501299, r s4880, rs2234693, rs1061622, rs6058017, rs7501331, rs223 2228** |

As another example, when the skin condition is elasticity, learning models may be constructed for elasticity determined using a Cutometer. SNPs used for evaluation of these skin conditions are shown below. One or more SNPs selected from the group consisting of the SNPs listed in the following tables can be used for learning model creation and for evaluation of skin condition (elasticity) using the learning model.

### [Table 11]

**Table 11: Elasticity**

| | |
|---|---|
| Skin11_linear_m1m2 | rs3829251, rs11234027, rs1030868, rs964184, rs1050450, r s2232228, rs4654748, rs1485766, rs1061622, rs2287074, rs 1800414, rs1107946, rs2227564, rs3785079, rs7201, rs3760 776, rs10515552, rs2987983, rs1540771, rs10741657, rs406 5, rs1501299, rs2241145, rs7799039, rs10882272, rs746533 30, rs2282679, rs12785878, rs1126643, rs7501331, rs12560 62, rs2108622, rs2246416, rs4880, rs2228479, rs2234693, rs2298585, rs1993116, rs1050565, rs12934922, rs3865188 |
| Skin11_upper_m1m2 | rs1107946, r810515552, rs1800414, rs964184, rs1485766, r s7201, rs10741657, rs11234027, rs1501299. rs2246416, rs3 865188, rs6810075, rs2010963, rs1540771, rs1030868 |
| Skin11_lower_m1m2 | rs3760776, rs4880, rs1030868, rs1485766, rs1050450, rs20 10963, rs3829251, rs2108622, rs2227564, rs2232228, rs105 0565, rs964184, rs2234693, rs17577, rs1800414, rs1088227 2, rs11234027, rs2987983, rs74653330, rs2298585, rs12785 878, rs1107946, rs12377462. rs12051272, rs1501299, rs750 1331, rs10741657, rs41281112, rs2241145, rs3785079, rs72 01, rs1126643, rs1047781, rs2282679, rs2246416, rs4065, rs2285053, rs3865188, rs1061622 |

As another example, when the skin condition is sebum amount, learning models may be constructed for measured sebum amounts determined using a Sebumeter. SNPs used for evaluation of these skin conditions are shown below. One or more SNPs selected from the group consisting of the SNPs listed in the following tables can be used for learning model creation and for evaluation of skin condition (sebum amount) using the learning model.

### [Table 12]

**Table 12: Sebum amount**

| | |
|---|---|
| Skin12_linear_m1m2 | rs1256062, rs74653330, rs2987983, rs1137101, rs3829251, rs6058017, rs2246416, rs17577, rs2234693, rs2046571, rs1 540771, rs4654748, rs3785079, rs2285053, rs2108622, rs81 10862, rs1667255, rs1030868, rs1126643, rs182052, rs1278 5878, rs833061, rs3865188, rs4065, rs1047781, rs10515552 , rs2287076, rs2228479, rs964184, rs2282679, rs6810075, rs1799750, rs11234027, rs1107946, rs9340799, rs7201, rs2 298585, rs2010963, rs12377462, rs2232228, rs1501299, rs1 1057830 |
| Skin12_upper_m1m2 | rs2246416, rs2046571, rs3829251, rs1256062, rs11234027, rs6058017, rs4880, rs1800414, rs1050565, rs1540771, rs10 515552, rs4654748, rs964184, r81799750, rs1126643, rs148 5766, rs4065, rs10741657, rs2060793, rs2282679, rs228505 3, rs3785079, rs2232228, rs2108622, rs11057830, rs811086 2, rs3865188, rs41303970, rs2987983. rs1030868, rs376077 6, rs12934922, rs1047781, rs17577, rs2234693, rs12377462 , rs1993116, rs833061, rs2227564, rs2287076, rs74653330 , rs1501299 |
| Skin12_lower_m1m2 | rs1256062, rs11057830, rs4880, rs8110862, rsl540771, rs2 108622, rs1047781, rs6058017, rs74653330, rs833061, rs20 10963, rs9340799, rs2287076, rs1801133, rs1501299, rs110 7946, rs2234693, rs2285053, rs7201, rs17577, rs4065 |

As another example, when the skin condition is moisture content (of the cheek or inner forearm), learning models may be constructed for measured moisture content values determined using a Corneometer and transepidermal water loss determined using a Vapometer. SNPs used for evaluation of these skin conditions are shown below. One or more SNPs selected from the group consisting of the SNPs listed in the following tables can be used for learning model creation and for evaluation of skin condition (moisture contents 1 and 2) using the learning model.

### [Table 13]

**Table 13: Moisture content 1, cheek**

| | |
|---|---|
| Skin13_linear_m1m2 | rs1801133, rs964184, rs2246416, rs2298585, rs1667255, rs 74653330, rs1126643, rs1540771, rs8110862, rs2282679, rs 2060793, rs12785878, rs1047781, rs3760776, rs7201, rs204 6571, rs1799750, rs1050565, rs11234027, rs2108622, rs222 7564, rs1485766, rs182052, rs1993116, rs2232228, rs12377 462, rs2287076, rs41281112, rs2241145, rs2987983, rs1074 1657, rs2010963, rs2285053, rs12051272, rs1137101, rs681 0075, rs17822931, rs2228479, rs1501299 |
| Skin13_upper_m1m2 | rs2108622, rs1667255, rs964184, rs7799039, rs1540771, rs 2298585, rs1047781, rs2246416, rs2285053, rs3829251, rs1 2785878, rs10741657, rs2987983, rs1137101, rs2228479, rs 4654748, rs6810075, rs1485766, rs2287076, rs1799750, rs9 340799, rs1801133, rs8110862, rs17822931, rs4880, rs1293 4922, rs1050565, rs3865188, rs2060793, rs1993116, rs1205 1272, rs41281112, rs1107946 |
| Skin13_lower_m1m2 | rs8110862, rs2234693, rs1800414, rs7201, rs2010963, rs12 377462, rs964184, rs1799724, rs2246416, rs74653330, rs11 234027, rs17822931, rs1799750, rs2046571, rs1485766, rs1 1057830, rs1050450, rs10741657, rs1126643, rs833061, rs1 2785878, rs1050565, rs2227564, rs8326, rs2232228, rs1051 5552, rs1667255, rs4065 |

### [Table 14]

**Table 14: Moisture content 2, inner upper arm**

| | |
|---|---|
| Skin14_linear_m1m2 | rs1799724, rs1799750, rs12377462, rs2241145, rs3785079, rs1050565, rs2282679, rs964184, rs1050450, rs1667255, rs 8326, rs17822931, rs74653330, rs2246416, rs9340799, rs11 234027, rs3829251, rs1047781, rs1800414, rs1501299, rs72 01, rs1107946, rs1801133, rs2227564, rs4065, rs2046571, rs833061, rs2010963, rs2285053, rs2298585, rs12785878, r s4880, rs1540771, rs7501331 |
| Skin14_upper_m1m2 | rs1050450, rs2046571, rs1799724, rs12377462, rs3785079, rs964184, rs2241145, rs4880, rs10741657, rs1047781, rs46 54748, rs2108622, rs3865188, rs4065, r81485766, rs112340 27, rs17822931, rs1126643, rs1050565, rs1667255, rs22826 79, rs12785878, rs3829251, rs1799750, rs1801133, rs29879 83, rs12934922, rs182052, rs2232228, rs41281112, rs11079 46, rs2228479, rs9340799, rs41303970, rs1540771, rs12560 62 |
| Skin14_lower_m1m2 | rs12785878, rs12934922, rs2227564, rs1667255, rs1256062 , rs3785079, rs1047781, rs12377462, rs1050565, rs4880, r s1107946, rs2241145, rs7501331, rs11234027. rs3829251, r s116643, rs2046571, rs1799750, rs7201, rs4654748, rs964 184, rs1800414, rs1801133, rs2108622 |

As another example, when the skin condition is barrier function (of the cheek or inner forearm), learning models may be constructed for transepidermal water loss (barrier function) determined using a Vapometer. SNPs used for evaluation of these skin conditions are shown below. One or more SNPs selected from the group consisting of the SNPs listed in the following tables can be used for learning model creation and for evaluation of skin condition (barriers 1 and 2) using the learning model.

### [Table 15]

**Table 15: Barrier, cheek**

| | |
|---|---|
| Skin15_linear_m1m2 | rs1107946, rs2287076, rs41281112, rs3865188, rs2046571, rsl540771, rs2287074, rs1030868, rs1485766, rs1667255, r s1801133, rs7799039, rs2246416, rs1061622, rs10882272, r s833061, rs964184, rs2108622, rs9340799, rs12051272, rs2 227564, rs2228479, rs6810075, rs3829251, rs10741657, rs2 060793, rs4065, rs1799750, rs2232228, rs2234693, rs81108 62, rs2241145, rs2285053, rs2282679, rs2298585, rs7201, rs1256062, rs1799724, rs1993116, rs12785878, rs3760776, rs1800414 |
| Skin15_upper_m1m2 | rs1050565, rs1540771, rs1107946, rs2298585, rs3785079, r s1667255, rs2232228, rs1485766, rs2046571, rs41303970, r s10882272, rs12051272, rs3865188, rs12785878, rs964184, rs2246416, rs1799750, rs2010963, rs17577, rs12377462, rs 2228479, rs2287076, rs2287074, rs1800414, rs2060793, rs4 880, rs1801133, rs7201, rs2241145, rs2285053, rs6810075 , rs1799724, rs10741657, rs1061622, rs9340799, rs2227564 |
| Skin15_lower_m1m2 | rs7799039, rs2228479, rs2285053, rs964184, rs2246416, rs 1501299, rs7501331, rs2287076, rs2282679, rs8110862, rs2 287074, rs1667255, rs11057830, rs833061, rs1485766, rs18 2052, rs4654748, rs7201, rs2241145, rs41281112, rs123774 62, rs12785878, rs2046571, rs11234027. rs3829251, rs3865 188, rs4065, rs2227564, rs41303970 |

### [Table 16]

**Table 16: Barrier, inner upper arm**

| | |
|---|---|
| Skin16_linear_m1m2 | rs2285053, rs833061, rs2282679, rs2298585, rs2010963, rs 1799724, rs1061622, rs964184, rs2287076, rs2108622, rs16 67255, rs1030868, rs4654748, rs1540771, rs1799750, rs224 6416, rs2241145, rs2232228, rs3865188, rs2287074, rs7501 331, rs11057830, rs2987983, rs12785878, rs1126643, rs746 53330, rs9340799, rs182052, rs3785079, rs2227564, rs1074 1657, rs1501299, rs2234693, rs12934922, rs12377462, rs18 01133, rs7799039, rs6810075, rs8326, rs1047781, rs17577 |
| Skin16_upper_m1m2 | rs2285053, rs1030868, rs2241145, rs12377462, rs2010963, rs2234693, rs1799750, rs1667255, rs2287074, rs2287076, r s1540771, rs8326, rs3865188, rs1126643, rs10882272, rs20 46571, rs182052, rs6810075, rs833061, rs1061622, rs17997 24, rs2282679, rs41303970, rs3785079, rs1050450, rs10477 81, rs12051272, rs8110862, rs1256062, rs12785878, rs9641 84, rs10741657, rs2232228 |
| Skin16_lower_m1m2 | rs2282679, rs2010963, rs12785878, rs833061, rs17577, rs2 228479, rs1107946, rs1256062, rs1540771, rs964184, rs106 1622, rs2246416, rs2232228, rs17822931, rs1501299, rs378 5079, rs74653330, rs4654748, rs2108622, rs1137101, rs107 41657, rs41303970, rs2227564, rs8110862, rs1667255 |

As another example, for eye corner wrinkle area, wrinkle volume and wrinkle maximum depth, learning models may be constructed for measured values in replica analysis. SNPs used for evaluation of these skin conditions are shown below. One or more SNPs selected from the group consisting of the SNPs listed in the following tables can be used for learning model creation and for evaluation of skin condition (wrinkle area, wrinkle volume and wrinkle maximum depth) using the learning model.

### [Table 17]

**Table 17: Wrinkle area**

| | |
|---|---|
| Skin21_linear_m1m2 | rs74653330, rs2298585, rs1061622, rs2282679, rs2108622, rs1540771, rs11057834, rs833061, rs1799724, rs2287074, r s2232228, rs3760776, rs1993116, rs7201, rs964184, rs1074 1657, rs12051272, rs2287076, rs12785878, rs1799750, rs16 67255, rs8326, rs1800414, rs12377462, rs1256062, rs14857 66, rs2285053, rs2228479, rs6810075, rs182052, rs1107946 , rs2246416, rs1501299, rs3829251, rs11234027, rs4880 |
| Skin21_upper_m1m2 | rs2108622, rs1540771, rs7201, rs1993116, rs2046571, rs22 85053, rs2298585, rs10741657, rs1801133, rs12377462, rs1 2785878, rs3785079, rs2227564, rs12934922, rs1050450, rs 1256062, rs17577, rs6810075, rs182052, rs1667255, rs1205 1272, rs4654748, rs11234027, rs2987983, rs964184, rs2287 076, rs3829251, rs1799750, rs2232228 |
| Skin21_lower_m1m2 | rs1540771, rs1799724, rs3760776, rs11057830, rs10741657 , rs2010963, rs182052, rs1061622, rs1256062, rs2987983, rs7201, rs3865188, rs74653330, rs1107946, rs6810075, rs2 246416, rs833061, rs2108622, rs2287074, rs1050450, rs123 77462, rs12785878, rs1126643, rs2298585, rs1993116, rs18 00414, rs2241145, rs2228479, rs3785079, rs2282679, rs222 7564, rs41281112, rs1485766, rs4065, rs1501299 |

### [Table 18]

**Table 18: Wrinkle volume**

| | |
|---|---|
| Skin22_linear_m1m2 | rs2298585, rs74653330, rs1061622, rsl540771, rs2282679, rs3785079, rs833061, rs2232228, rs2108622, rs11057830, r s1799724, rs3760776, rs1799750, rs1256062, rs182052, rs3 829251, rs964184, rs7201, rs2287074, rs8326, rs11234027 , rs2287076, rs10515552, rs12377462, rs1667255, rs120512 72, rs2285053, rs1485766, rs8110862, rs1993116, rs681007 5, rs2987983, rs12785878, rs10741657, rs2246416, rs11079 46, rs2010963, rs2228479, rs1800414 |
| Skin22_upper_m1m2 | rsl540771, rs3785079, rs2010963, rs2108622, rs7201, rs83 26, rs833061, rs1993116, rs2298585, rs1107946, rs1074165 7, rs1801133, rs964184, rs1799724, rs182052, rs1061622, rs11057830, rs41303970, rs1800414, rs2287074, rs2287076 , rs3829251, rs6810075, rs2241145, rs2232228, rs1501299 , rs4654748, rs12785878, rs11234027, rs12051272 |
| Skin22_lower_m1m2 | rsl540771, rs2298585, rs1799724, rs2227564, rs1061622, r s12785878, rs2246416. rs2282679, rs2010963, rs2108622, r s11057830, rs1256062, rs833061, rs2241145, rs182052, rs1 050450, rs3865188, rs6810075, rs1485766, rs7201, rs18004 14, rs3760776, rs1126643, rs2232228, rs2987983, rs779903 9, rs1137101, rs1107946, rs17822931, rs964184, rs1074165 7, rs9340799, rs1993116, rs8110862, rs12051272, rs223469 3, rs8326 |

### [Table 19]

**Table 19: Maximum wrinkle depth**

| | |
|---|---|
| Skin23_linear_m1m2 | rs1667255, rs10515552, rs1801133, rs2108622, rs833061, r s11057830, rs3785079, rs964184, rs2010963, rs1107946, rs 4654748, rs2287076, rs2285053, rs1485766, rs1800414, rs1 799724, rs2241145, rs2298585, rs3760776, rs8326, rs10616 22, rs1126643, rs10741657, rsl540771, rs2287074, rs41281 112, rs2282679, rs74653330, rs3829251, rs1050565, rs1047 781, rs7201, rs12377462, rs182052, rs11234027, rs2228479 , rs1137101, rs17577, rs2227564, rs6058017, rs2987983. r s1256062, rs2046571 |
| Skin23_upper_m1m2 | rs1800414, rs1107946, rs1137101, rs2227564, rs2108622, r s7201, rs1801133, rs11057830, rs964184, rs8326, rs378507 9, rs2285053, rs1485766, rs1256062, rs1061622, rs1051555 2, rs2241145, rs1667255, rs2228479, rs3760776, rs1540771 , rs1126643, rs12377462, rs833061, rs41303970, rs3865188 , rs8110862, rs2987983, rs10741657, rs2287074, rs1799750 , rs2246416, rs1501299, rs10882272 |
| Skin23_lower_m1m2 | rs1540771, rs182052, rs2287074, rs833061, rs2046571, rs2 287076, rs2228479, rs1799724, rs1061622, rs41281112, rs9 64184, rs2232228, rs2298585, rs12377462, rs9340799, rs29 87983, rs1050450, rs8326, rs7201, rs2234693, rs1801133, rs6810075, rs1107946, rs1126643, rs2282679, rs12934922, rs3785079, rs1800414, rs2241145, rs3760776, rs3865188, r s4880, rs11057830, rs1047781, rs8110862, rs3829251 |

Another aspect of the invention relates to a skin evaluating method using individual SNP information selected from the group consisting of the following: rs1800629, rs2108622, rs1047781, rs12203592, rs16891982, rs3760776, rs9340799, rs12913832, rs17822931, rs964184, rs18o1133, rs2228479, rs10515552, rs10741657, rs10882272, rs11057830, rs11234027, rs12272004, rs12377462, rs12785878, rs12931267, rsl540771, rs1667255, rs1993116, rs2060793, rs2227564, rs2282679, rs2298585, rs3829251, rs41281112, rs4654748, rs492602, rs602662, rs1030868, rs1126643, rs1256062, rs12934922, rs1485766, rs1501299, rs17577, rs1799724, rs18,00012, rs1800414, rs2010963, rs2234693, rs2241145, rs2285053, rs2287074, rs2287076, rs2987983, rs3918242, rs4065, rs4252125, rs6152, rs7201, rs74653330, rs7501331, rs8110862, rs8326, rs833061, rs1050565, rs1799750, rs4880, rs1050450, rs6058017, rs1061622, rs2046571, rs2232228, rs3785079, rs2246416, rs1107946, rs11568737, rs41303970, rs12051272, rs182052, rs3865188, rs6810075, rs7799039, rs1137101, and at least one item of environmental factor information selected from the group consisting of age, body height, body weight, BMI, ultraviolet exposure information and smoking information,
wherein the skin evaluating method includes determining skin condition by weighting the SNP information and environmental factor information for contribution to skin condition. The SNP used may be appropriately selected depending on the skin condition to be evaluated. More specifically, the relationships between skin conditions to be evaluated and SNPs to be used are as listed in Tables 1 to 19.

For the purpose of the invention, a SNP is represented as an "rs" number (Reference SNP ID number). Detailed SNP information (position on chromosome and mutations) corresponding to each rs number is managed by the National Center for Biotechnology Information (NCBI) and can be found at the NCBI website (http://www.ncbi.nlm.nih.gov/).

Detection of SNPs may be by any publicly known method. As an example, nucleic acid of a test subject is purified from a biological sample of the test subject, such as saliva, blood, mucous membrane, tissue strip or hair, using an established method, and a SNP is detected in the purified nucleic acid. The method for determining skin properties according to the invention may therefore include a sample preparation step and a nucleic acid purification step. The nucleic acid may be DNA or RNA, and in the case of RNA the purification is preferably followed by reverse transcription and preparation of DNA. In the SNP detection step, any method may be used that is able to detect SNPs in purified nucleic acid. The area surrounding the SNP of interest may be sequenced to detect the SNP, or detection may be using a PCR-based method, a DNA probe method or a mass spectrometry method. PCR-based methods include SNP typing methods, TaqMan PCR methods, single nucleotide extension methods, Pyrosequencing and Exonuclease Cycling Assay. DNA probe methods include the Invader method (Comprehensive gene polymorphism analysis (SNP), Nippon Yakurigaku Zasshi, 125, 148-152, 2005). SNPs are often accompanied by other SNPs in linkage disequilibrium. A target SNP can be detected by detecting a SNP that is in linkage disequilibrium with the target SNP. For the purpose of the invention, therefore, SNP detection is not limited to direct detection of the target SNP but also includes detection of the target SNP by detection of SNPs in linkage disequilibrium with it.

The presence of a SNP can also be detected based on the nucleotide sequence of an individual that has already been sequenced. In this case, the presence of a SNP can be detected by examining the sequence at the position where the SNP exists among the already sequenced nucleotide sequence data. SNP detection in this case may be carried out by inputting nucleotide sequence data into a computer in which SNP information is stored. According to a more preferred aspect, the input is through a terminal to the internet, the SNP is detected on a server, and the detection results are outputted from the internet to the terminal.

The input unit 12 includes an interface. The interface may be connected to an operating unit such as a keyboard or mouse, a communication unit, and an external memory unit such as a CD-ROM, DVD-ROM, BD-ROM or memory stick. SNP information and environmental factor information may be inputted through the input unit and stored in the storage unit. Genetic sequence information for the subject may also be inputted through the input unit instead of SNP information. The inputted sequence information may first be stored in the storage unit, or it may be sent directly to the processing unit. The genetic sequence information of the subject may be the full genome sequence of the subject, or it may be only a partial target sequence or only SNP information. Instructions for processing at the processing unit can be given through the input unit via an operating unit.

The output unit 15 includes an interface. The interface outputs a skin type or skin condition outputted by the learning device. SNP information relating to the skin type may also be outputted at the learning device in addition to the skin type or skin condition. The output unit may be output means such as a display device with a liquid crystal display that directly displays the results of the learning system, or a printer, or it may be a communication unit for output to an external memory unit or output to a network.

The communication unit can also function as an input unit and/or output unit for a communication interface such as a LAN or port for connection of the information processing device to a network.

The storage unit 11 has a portable storage device which may be a memory device such as a RAM, ROM or flash memory, a fixed disk device such as a hard disk drive, or a flexible disk or optical disk. The storage unit stores data and instructions inputted through the input unit, and the processing results of the learning system, as well as the program, database and output forms used for processing by a computer. The computer program may be a computer readable recording medium such as a CD-ROM or DVD-ROM, or it may be installed via the internet. The computer program is installed in the storage unit using a commonly known setup program, for example.

The processing unit 14 controls an information processing device so that it inputs SNP information and environmental factor information inputted through the input unit into the learning system through an input acquisition unit, and acquires the skin type and skin condition outputted from the learning system via the communication unit. The processing unit executes computation processing according to the program stored in the storage unit. Computation processing is carried out by a processor inside the processing unit. The processor includes a functional module that controls the input unit, storage unit, communication unit and output unit, and is able to control various control operations. Each of the units may be constructed by independent integrated circuits, microprocessors and firmware. Information for the presence of SNPs detected by the processing unit is first stored in the storage unit. When genetic sequence information of a subject has been inputted instead of SNP information, the processing unit can operate to detect the presence of a SNP in the genetic sequence information. More specifically, the processing unit can detect the presence of a SNP among sequence information for a subject from sequence information of the SNP prestored in the storage unit, and sequence information for the subject that has been inputted through the input unit. By comparing the sequence information for the SNP stored in the storage unit and the sequence information for the subject that has been inputted through the input unit, or detecting the sequence information for the SNP that has been stored in the storage unit among the sequence information of the subject that has been inputted through the input unit, it is possible to detect the presence of the SNP.

Using a known statistical analysis technique or machine learning technique, the learning unit 13 learns relationships between environmental information and genetic information for SNPs, and previously measured skin condition measurement data. According to the invention, the learning model is constructed by selecting variables using Elastic Net, and using generalized linear model (logistic regression, multiple regression).

Yet another aspect of the invention relates to a method and computer for determining a skin property, as well as a program or method for controlling the computer, based on the presence of one or more SNPs selected from the group consisting of the following: rs1800629, rs2108622, rs1047781, rs12203592, rs16891982, rs3760776, rs9340799, rs12913832, rs17822931, rs964184, rs1801133, rs2228479, rs10515552, rs10741657, rs10882272, rs11057830, rs11234027, rs12272004, rs12377462, rs12785878, rs12931267, rs1540771, rs1667255, rs1993116, rs2060793, rs2227564, rs2282679, rs2298585, rs3829251, rs41281112, rs4654748, rs492602, rs602662, rs1030868, rs1126643, rs1256062, rs12934922, rs1485766, rs1501299, rs17577, rs1799724, rs18,00012, rs1800414, rs2010963, rs2234693, rs2241145, rs2285053, rs2287074, rs2287076, rs2987983, rs3918242, rs4065, rs4252125, rs6152, rs7201, rs74653330, rs7501331, rs8110862, rs8326, rs833061, rs1050565, rs1799750, rs4880, rs1050450, rs6058017, rs1061622, rs2046571, rs2232228, rs3785079, rs2246416, rs1107946, rs11568737, rs41303970, rs12051272, rs182052, rs3865188, rs6810075, rs7799039 and rs1137101. One or more selected from the group consisting of rs1047781, rs1050450, rs1137101, rs12203592, rs12934922, rs182052, rs2287076, rs3918242, rs41303970, rs4252125, rs602662, rs6152 and rs6810075 may also be used among these listed SNPs.

More specifically, the method of determining a skin property includes:
a step of detecting the one or more SNPs, and
a step of determining a skin property based on the detected SNP.

More specifically, the computer for determining a skin property includes:
a storage unit in which information relating to at least one SNP and information for a skin property relating to the SNP are stored,
an input unit that inputs DNA information of a user,
a processing unit that detects the presence of the stored SNP among the inputted DNA information, and
an output unit that outputs the information for the skin property associated with the detected SNP.

More specifically, the program or method for controlling a computer that determines a skin property includes:
causing a storage unit to store information relating to at least one SNP and information for a skin property relating to the SNP,
causing input of DNA information of a user through an input unit,
causing a processing unit to detect the presence of the stored SNP among the inputted DNA information, and
causing an output unit to output the information for the skin property associated with the detected SNP. Another aspect of the invention relates to a storage medium that stores the control program.

The computer of the invention may also be a skin property-determining device. The evaluation system or computer of the invention may also form part of a system that includes a server and terminal device. When a server and terminal device are included, the input unit and output unit are each connected to the network via an interface unit or communication unit. In this case, genetic information and/or environmental information of the user are inputted through a separate terminal device connected to the network and processed at a server, and a skin condition or skin property is outputted to the same or a different terminal device. Genetic information of the user may be provided as genomic information or partial genomic sequence information, or it may be provided as the specified SNP information alone. When all or partial genomic information has been provided, the presence of a SNP is detected at the processing unit of the server, based on a SNP stored in the storage unit. In this system, several servers may be distributed on a network so that a service is provided in the form of cloud computing, with collaboration between each of the servers to share processing by the processing unit or learning unit. This will allow more efficient processing by the evaluation system.

A skin property may be selected from the group consisting of skin morphology, skin quality and skin color properties. Skin morphology may be selected from among wrinkles, texture and firmness. Skin quality may be selected from among sebum amount, moisture content and skin barrier function. Skin color properties may be selected from among skin ground color, skin color at ultraviolet-exposed sections, pigmented spots, brightness and yellowness. A cosmetic, supplement and/or beauty method can be provided based on a skin property determined by the method of determining a skin property or a computer that determines a skin property, according to the invention.

A skin property can be determined by detection of a single SNP, but detection of multiple SNPs to determine a skin property is preferred from the viewpoint of achieving higher sensitivity. As an example for determining a skin property based on detection of multiple SNPs, it is determined whether the SNP has a positive or negative action on the skin property, and points are assigned based on its presence to calculate a SNP score. By determining in advance the relationship between SNP score and skin property, it is possible to determine a skin property based on a SNP score.

The skin property to be determined may be one or more selected from the group consisting of skin morphology, skin quality and skin color properties. In the relationship between SNPs and these skin properties, the heterozygosity or homozygosity of the SNP may have a negative function or a positive function on the skin properties. The SNP score can be calculated based on the number of SNPs with negative function and the number of SNPs with positive function. As an example, the SNP score can be calculated by subtracting the number of SNPs with negative function from the number of SNPs with positive function. As another example, conversely, the SNP score can be calculated by subtracting the number of SNPs with positive function from the number of SNPs with negative function. The relationship between a skin property and SNP score may be determined in advance and the skin property of a test subject may be determined based on a calculated SNP score.

When a skin property has been determined by the skin property determining method of the invention, counseling may be provided based on the information for the skin property. One aspect of the invention, therefore, relates to a counseling method. In counseling, advice may be provided on lifestyle habits or selection of cosmetics or supplements to be used. As an example of skin color properties, the amount of melanin on the upper arm is related to the original skin ground color of a person, while the amount of melanin on the face is related to skin color affected by ultraviolet rays. Therefore, SNP analysis can determine that a subject that has an increased amount of melanin in the face while tending to have a low amount of melanin in the upper arm, has a whiter color which is more susceptible to the effects of ultraviolet rays, and may therefore need more care against ultraviolet rays. For such a subject, a lifestyle habit of avoiding ultraviolet rays may be proposed while also suggesting the use of cosmetics such as strong protective agents against ultraviolet rays.

When a tendency for wrinkles has been assessed, a cosmetic or supplement may be provided that contains components that are able to augment production of collagen fibers or elastic fibers such as collagen or elastin, and/or components such as matrix metalloproteinase inhibitors that can inhibit decomposition of elastic fibers. Such components include extracellular matrix decomposition inhibitors such as matrix metalloproteinase inhibitors, or more specifically, collagenase inhibitors. Specific components include retinol, winged-bean extract and mangosteen extract (Keshouhin no Yuyosei, Hyokagijutsu no Shinpo to Shouraitenbou, Yakuji Nippo, (2001), Section 7, Wrinkle cosmetics, pp.162-177).

When a tendency for skin color problems such as pigmented spots has been assessed, a cosmetic or supplement may be provided that contains components having UV blocking action, melanin formation-inhibiting action and/or keratin turnover accelerating action. Examples of such components include UV protective agents, vitamin C derivatives, hydroquinone, tranexamic acid, potassium 4-methoxysalicylate salt, arbutin, kojic acid, lucinol and vitamin C derivatives (Keshouhin no Yuyosei, Hyokagijutsu no Shinpo to Shouraitenbou, Yakuji Nippo, (2001), Section 6, Whitening cosmetics, pp.144-161). A suitable cosmetic base or cosmetic may be proposed, or a makeup method may be proposed.

When reduction of skin function such as texture, firmness, sebum, moisture content or skin barrier function has been assessed, a cosmetic or supplement may be provided which contains components that increase skin function. Texture- or firmness-improving agents, sebum-reducing agents, moisture content increase accelerators and skin barrier function enhancers are known. Examples of such components include humectants, tranexamic acid and winged-bean extract.

Another aspect of the invention relates to a kit for determining a skin property. The kit includes a nucleic acid reagent for detection of at least one SNP selected from the group consisting of the following: rs1800629, rs2108622, rs1047781, rs12203592, rs16891982, rs3760776, rs9340799, rs12913832, rs17822931, rs964184, rs1801133, rs2228479, rs10515552, rs10741657, rs10882272, rs11057830, rs11234027, rs12272004, rs12377462, rs12785878, rs12931267, rs1540771, rs1667255, rs1993116, rs2060793, rs2227564, rs2282679, rs2298585, rs3829251, rs41281112, rs4654748, rs492602, rs602662, rs1030868, rs1126643, rs1256062, rs12934922, rs1485766, rs1501299, rs17577, rs1799724, rs18,00012, rs1800414, rs2010963, rs2234693, rs2241145, rs2285053, rs2287074, rs2287076, rs2987983, rs3918242, rs4065, rs4252125, rs6152, rs7201, rs74653330, rs7501331, rs8110862, rs8326, rs833061, rs1050565, rs1799750, rs4880, rs1050450, rs6058017, rs1061622, rs2046571, rs2232228, rs3785079, rs2246416, rs1107946, rs11568737, rs41303970, rs12051272, rs182052, rs3865188, rs6810075, rs7799039 and rs1137101, or a SNP in linkage disequilibrium with it. The kit may also include a nucleic acid extraction reagent and purification reagent. It may also include an instruction manual explaining the relationship between a detected SNP and skin condition. One or more selected from the group consisting of rs1047781, rs1050450, rs1137101, rs12203592, rs12934922, rs182052, rs2287076, rs3918242, rs41303970, rs4252125, rs602662, rs6152 and rs6810075 may also be used among these listed SNPs.

Examples of such nucleic acid reagents include primers capable of selectively amplifying the SNP, DNA polymerase, dNTP and optionally amplification buffer. Primers capable of amplifying SNPs can be designed according to each SNP.

When a nucleic acid reagent uses a nucleic acid probe, the kit may also include the probe detection reagent. Such nucleic acid probes may be free probes or probes immobilized on a DNA chip for simultaneous detection of multiple SNPs. Such nucleic acid probes may also be labeled to allow detection of specific SNPs.

All of the publications mentioned throughout the present specification are incorporated herein in their entirety by reference.

The examples of the invention described below are intended to serve merely as illustration and do not limit the technical scope of the invention. The technical scope of the invention is limited solely by the description in the Claims. Modifications of the invention, such as additions, deletions or substitutions to the constituent features of the invention, are possible so long as the gist of the invention is maintained.

### EXAMPLES

### Example 1: Analysis subject

The subjects were female volunteers aged 20 to 79. Before analysis, a questionnaire survey was taken regarding age, body height, body weight, BMI (calculated from body height and body weight by an established method), ultraviolet exposure information and smoking information. For ultraviolet exposure information, the survey classified current awareness of tanning on 4 levels (two levels of tanning awareness, two levels of tanning non-awareness), and for more detailed description of measures taken against ultraviolet rays, it classified tanning countermeasures taken from birth until the current time (age 1-14, age 15-19, age 20-24, age 25-29 and in 10-year units thereafter) on 3 levels (no active ultraviolet ray countermeasures, ultraviolet ray countermeasures on strong sunlit days, ultraviolet ray countermeasures even on weak sunlit days). The survey divided smoking information into 3 groups: no history of smoking, past history of smoking 20 or more cigarettes per day, and past history of smoking less than 20 cigarettes per day.

### Example 2: Acquisition of skin measurement data for analysis subjects

Skin property values measured for female volunteers aged 20 to 79 as subjects were skin wrinkle conditions, pigmented spot condition, skin color (cheek melanin amount, base skin (inner upper arm) melanin amount, brightness, yellowness) and skin condition (barrier function, sebum amount, firmness, texture). For the measurements, wrinkle condition and pigmented spot condition were determined by calculating index values for wrinkles or pigmented spots by a special method from a captured image using Visia Evolution (Canfield Scientific), while for pigmented spots the number of pigmented spots and pigmented spot area were also calculated using a skin imaging device. Skin color was analyzed using a CM-700d spectrophotometer (Konica Minolta Holdings, Inc.), and the cheek melanin amount, brightness (L* • measured value) and yellowness (b* • measured value) were calculated. Skin color on the inner upper arm, as a site not exposed to sunlight, was also measured and the melanin amount was calculated. Special commercial devices were used for skin condition measuring the stratum corneum moisture content (Corneometer), transepidermal water loss (barrier function) (Vapometer), sebum amount (Sebumeter) and firmness (Cutometer), while the texture condition was measured using the original measuring device Skinvisiom, and special analysis software was used to determine the degree of disturbance in texture. Eye corner wrinkles were measured three-dimensionally, creating a replica of the skin surface with silicone rubber and loading the data into a computer, performing image processing with a private analysis system, and calculating the depth, volume and area of wrinkles.

### Example 3: Determining SNP of analysis subject

The following 79 different SNPs, expected to affect skin property based on past dermatologic observations, were selected for analysis subjects rs1800629, rs2108622, rs1047781, rs12203592, rs16891982, rs3760776, rs9340799, rs12913832, rs17822931, rs964184, rs1801133, rs2228479, rs10515552, rs10741657, rs10882272, rs11057830, rs11234027, rs12272004, rs12377462, rs12785878, rs12931267, rs1540771, rs1667255, rs1993116, rs2060793, rs2227564, rs2282679, rs2298585, rs3829251, rs41281112, rs4654748, rs492602, rs602662, rs1030868, rs1126643, rs1256062, rs12934922, rs1485766, rs1501299, rs17577, rs1799724, rs18,00012, rs1800414, rs2010963, rs2234693, rs2241145, rs2285053, rs2287074, rs2287076, rs2987983, rs3918242, rs4065, rs4252125, rs6152, rs7201, rs74653330, rs7501331, rs8110862, rs8326, rs833061, rs1050565, rs1799750, rs4880, rs1050450, rs6058017, rs1061622, rs2046571, rs2232228, rs3785079, rs2246416, rs1107946, rs11568737, rs41303970, rs12051272, rs182052, rs3865188, rs6810075, rs7799039 and rs1137101.

The selection criteria selected were factors, enzymes and extracellular matrix proteins functioning in the epidermis, basal membrane, dermis, subcutaneous fatty tissue and systemically (hormones and vitamins), and genes with SNPs were selected among them as candidates. These SNPs are considered to potentially have effects on different skin traits.

Next, 4 different genetic models, a recessive model, dominant model, additive model and multiplicative model, were designed for each of the 79 SNPs, and SNPs for the total of 316 different genetic models of the 79 SNPs were used as genetic information. Using the genetic information and environmental information as explanatory variables, variable selection was carried out using Elastic Net and optimal genetic models were determined for each SNP. Determining a genetic model before creating a learning model according to the invention is expected to increase precision for determining skin type based on SNPs.

Saliva was used as the sample for determining SNPs for the analysis subjects. The saliva was sampled using Oragene^{R} DNA OG-500 (DNA Genotek Inc.) and the DNA was stabilized. DNA was purified from the saliva and a DNA array was used to determine SNPs, acquiring information for the 79 different pre-selected SNPs.

### Example 4: Creation of learning model for determining skin condition score, using measured values for a skin condition as response variables

Multiple regression analysis was performed using measured values for a skin condition selected from the group consisting of wrinkles, pigmented spots, cheek melanin amount, base skin (inner upper arm) melanin amount, brightness, yellowness, barrier function, sebum amount, firmness, texture, wrinkle area, wrinkle volume and wrinkle maximum depth, as response variables and using genetic information for the SNPs and environmental factor information including age, body height, body weight, BMI, ultraviolet exposure information and smoking information as explanatory variables, to create a learning model for determining skin condition score (Fig. 2). The number of SNPs used was n for creation of the learning model. Models were created for n = 1 to 4 and 5 to 10. In the learning model for determining skin condition score, a constant term was defined for each explanatory variable, and an explanatory variable was inputted to allow output of a skin condition as the response variable. One or more of the explanatory variables (for example, age, ultraviolet exposure information, smoking information, etc.) was changed to allow prediction of change in future skin condition.

### Example 5: Classification into high value group and non-high value group (or low value group and non-low value group), and creation of learning model for assessing skin type with classification result as response variable

Based on a measured skin condition value selected from the group consisting of wrinkles, pigmented spots, cheek melanin amount, base skin (inner upper arm) melanin amount, brightness, yellowness, barrier function, sebum amount, firmness and texture, a high value group and low value group for the skin condition was determined for each age from the respective distributions of the measured values. Specifically, in the case of a high value group model for assessing tendency for a skin type (for example, tendency for pigmented spots if the subject skin type was pigmented spots), 1 was defined for a measured value for skin obtained by skin examination that was in the upper 25% of the total data for each age (split into 5-year groups), and 0 was defined for all other cases (non-high value group) (Fig. 3). In the case of a low value group model for assessing tendency against a skin type (for example, tendency against pigmented spots if the subject skin type was pigmented spots), 1 was defined for a measured value for skin obtained by skin examination that was in the lower 25% of the total data for each age (split into 5-year groups), and 0 was defined for all other cases (Fig. 3).

With 1 as the high value group and 0 as the non-high value group, in the case of a high value group assessment model, as response variables, and inputting information for the 79 different SNPs and environmental factor information based on questionnaire results as explanatory variables, logistic regression analysis was performed to create a learning model for assessing skin type. The number of SNPs used was n for creation of the learning model. Models were created for n = 1 to 4 and 5 to 10. In the learning model for assessing skin type, a constant term is defined for each explanatory variable, an explanatory variable is inputted to allow output of the tendency for the skin type (possibility of assignment to the high value group of the skin condition) and/or tendency against the skin type (possibility of assignment to the low value group of the skin condition), as the response variable.

### Example 6: Validation of learning model

For creation of the learning model, the dataset for an analysis subject was divided into 80% of the dataset as the learning dataset and 20% of the dataset as the validation dataset. One hundred different cross-validation datasets comprising a learning dataset and validation dataset were thus created. A learning model for determining skin condition score and a learning model for assessing skin type were constructed by the procedure described for Examples 4 and 5, and 100 different learning models were created as a result. The SNPs used in each learning model created for each skin condition were as follows. In the tables, the SNPs used in the 100 learning models for determining skin condition score are listed under the "linear" column. The SNPs used in the 100 learning models for assessing skin type for determining tendency for skin type (possibility of assignment to the high value group of the skin condition) are listed under the "upper" column. The SNPs used in the 100 learning models for assessing skin type for determining tendency against skin type (possibility of assignment to the low value group of the skin condition) are listed under the "lower" column.

### [Table 20]

**Table 1: Pigmented spots 1 (Visia pigmented spot index)**

| | |
|---|---|
| Skin01_linear_m1m2 | rs1030868, rs1050450, rs10741657, rs10882272, rs1107946, rs1126643, rs12051272, rs1256062, rs12785878, rs1501299, rs1540771, rs1799724, rs1799750, rs1800414, rs1801133, rs 2010963, rs2046571, rs2060793, rs2108622, rs2228479, rs22 34693, rs2246416, rs2282679, rs2285053, rs2287074, rs2287 076, rs2987983, rs3760776, rs3785079, rs4065, rs4654748, rs6058017, rs7201, rs74653330, rs8110862, rs8326, rs83306 1, rs964184 |
| Skin01_upper_m1m2 | rs1030868, rs1050450, rs10515552, rs10741657, rs10882272 , rs11057830, rs1107946, rs12377462, rs1256062, rs129349 22, rs1485766, rs1501299, rs1540771, rs1800414, rs199311 6, rs2010963, rs2046571, rs2060793, rs2108622, rs2227564 , rs2234693, rs2241145, rs2246416, rs2285053, rs2287074 , rs2287076, rs2987983, rs3785079, rs3829251, rs3865188 , rs4654748, rs7201, rs74653330, rs7501331, rs8110862, r s8326, rs833061, rs964184 |
| Skin01_lower_m1m2 | rs1047781, rs1050450, rs10741657, rs1107946, rs1137101, rs12051272, rs1256062, rs12934922, rs1485766, rs1667255 , rs1799724, rs1799750, rs1801133, rs182052, rs2010963, rs2046571, rs2108622, rs2234693, rs2246416, rs2285053, r s2287074, rs2287076, rs2987983, rs3760776, rs3865188, rs 6810075, rs7201, rs74653330, rs7799039, rs8110862, rs833 061, rs9340799, rs964184 |

### [Table 21]

**Table 2: Pigmented spots 2 (pigmented spot count)**

| | |
|---|---|
| Skin02_linear_m1m2 | rs1030868, rs1061622, rs10741657, rs11057830, rs1107946 , rs1126643, rs12051272, rs12377462, rs1256062, rs148576 6, rs1501299, rs1540771, rs1799750, rs1800414, rs1801133 , rs182052, rs2046571, rs2060793, rs2108622, rs2234693, rs2282679, rs2285053, rs2287074, rs2287076, rs2298585, r s2987983, rs3785079, rs3865188, rs4065, rs4880, rs681007 5, rs7201, rs74653330, rs7799039, rs8110862, rs833061 |
| Skin02_upper_m1m2 | rs10741657, rs1107946, rs1137101, rs12051272, rs1256062 , rs1485766, rs1667255, rs1799724, rs1799750, rs182052, rs2046571, rs2060793, rs2234693, rs2246416, rs2282679, r s2287074, rs2287076, rs2987983, rs3785079, rs3865188, rs 41303970, rs6810075, rs7201, rs833061, rs9340799 |
| Skin02_lower_m1m2 | rs1030868, rs1047781, rs1050450, rs1061622, rs10741657, rs11057830, rs1107946, rs11234027, rs12377462, rs1256062 , rs12785878, rs1540771, rs1667255, rs1799750, rs1801133 , rs182052, rs2010963, rs2046571, rs2060793, rs2108622, rs2282679, rs2285053, rs2987983, rs4065, rs4880, rs68100 75, rs7201, rs8110862, rs8326, rs833061, rs9340799 |

**Table 3: Pigmented spots 3 (pigmented spot area)**

| | |
|---|---|
| Skin03_linear_m1m2 | rs1061622, rs11057830, rs1107946, rs11234027, rs1126643 , rs1137101, rs12051272, rs12377462, rs1256062, rs127858 78, rs12934922, rs1485766, rs1540771, rs1667255, rs17822 931, rs1799750, rs1800414, rs2046571, rs2108622, rs22275 64, rs2234693, rs2241145, rs2246416, rs2282679, rs229858 5, rs3785079, rs3829251, rs3865188, rs4880, rs6058017, r s6810075, rs74653330, rs8110862, rs8326 |
| Skin03_upper_m1m2 | rs1030868, rs1047781, rs1061622, rs10882272, rs11234027, rs1137101, rs12377462, rs12785878, rs12934922, rs1501299, rs1540771, rs17577, rs1799750, rs1800414, rs2010963, rs20 46571, rs2227564, rs2234693, rs2241145, rs2246416, rs2282 679, rs2285053, rs2287074, rs2287076, rs2987983, rs376077 6, rs3785079, rs3829251, rs4654748, rs6058017, rs7201, rs 833061, rs9340799, rs964184 |
| Skin03_lower_m1m2 | rs1047781, rs1050565, rs1061622, rs10741657, rs1107946, r s11234027, rs1126643, rs1137101, rs12051272, rs12785878, rs1485766, rs1667255, rs1801133, rs2046571, rs2108622, rs 2227564, rs2228479, rs2234693, rs2241145, rs2246416, rs22 82679, rs2285053, rs2287074, rs2987983, rs3785079, rs3865 188, rs41281112, rs4880, rs6058017, rs74653330, rs8110862 , rs8326, rs833061, rs964184 |

### [Table 23]

**Table 4: Skin color 1 melanin amount, cheek)**

| | |
|---|---|
| Skin04_linear_m1m2 | rs1030868, rs1047781, rs10741657, rs1107946, rs1126643, rs1137101, rs12377462, rs1256062, rs12785878, rs1485766 , rs1540771, rs1799750, rs1800414, rs1993116, rs2010963 , rs2060793, rs2108622, rs2227564, rs2232228, rs2234693 , rs2241145, rs2246416, rs2282679, rs2285053, rs2287074 , rs2287076, rs2987983, rs3760776, rs3785079, rs3829251 , rs4065, rs4654748, rs4880, rs6058017, rs7201, rs746533 30, rs7501331, rs964184 |
| Skin04_upper_m1m2 | rs1030868, rs1047781, rs10741657, rs10882272, rs1107946 , rs12051272, rs12377462, rs12785878, rs12934922, rs1485 766, rs1540771, rs1799750, rs1800414, rs1993116, rs20109 63, rs2046571, rs2108622, rs2227564, rs2228479, rs223222 8, rs2234693, rs2241145, rs2246416, rs2282679, rs2287074 , rs2287076, rs2298585, rs3760776, rs3785079, rs3865188 , rs4880, rs6058017, rs7201, rs74653330, rs8110862, rs96 4184 |
| Skin04_lower_m1m2 | rs1047781, rs1050450, rs1050565, rs10515552, rs10741657 , rs1107946, rs1126643, rs12377462, rs1256062, rs1278587 8, rs1501299, rs1540771, rs17577, rs1799724, rs1800414, rs1801133, rs1993116, rs2010963, rs2046571, rs2060793, r s2108622, rs2227564, rs2232228, rs2234693, rs2241145, rs 2246416, rs2287074, rs2287076, rs3760776, rs3785079, rs3 829251, rs4065, rs41281112, rs41303970, rs4880, rs605801 7, rs7201, rs74653330, rs8326, rs9340799 |

### [Table 24]

**Table 5: Skin color 2 (melanin amount, inner upper arm)**

| | |
|---|---|
| Skin05_linear_m1m2 | rs74653330, rs1800414, rs1540771, rs1126643, rs2060793, rs1047781, rs2987983, rs7501331, rs2010963, rs1993116, r s11234027, rs6058017, rs9340799, rs2287074, rs2246416, r s3829251, rs10741657, rs3785079, rs2234693, rs11057830, rs182052, rs1799750, rs6810075, rs12051272, rs1501299, r s1485766, rs2285053, rs2282679, rs12934922, rs8110862, r s10882272 |
| Skin05_upper_m1m2 | rs74653330, rs7501331, rs2010963, rs1540771, rs1800414, rs9340799, rs2234693, rs182052, rs3829251, rs6810075, rs 2246416, rs12785878, rs10741657, rs2241145, rs1126643, r s6058017, rs2285053, rs3785079, rs964184, rs833061, rs12 56062, rs1799750, rs1047781, rs2046571, rs1501299, rs206 0793, rs11057830, rs2282679, rs1137101, rs7201, rs4880, rs11234027, rs1485766, rs12051272, rs1993116, rs1801133 , rs2108622 |
| Skin05_lower_m1m2 | rs74653330, rs2234693, rs9340799, rs1800414, rs1540771, rs1126643, rs3760776, rs8110862, rs2232228, rs2282679, r s964184, rs2228479, rs10741657, rs2060793, rs3785079, rs 7501331, rs1501299, rs2298585, rs2987983, rs2241145, rs1 2934922, rs1047781, rs1107946, rs2010963, rs1061622, rs1 2377462, rs2046571, rs1050565, rs1799724, rs1256062, rs1 993116, rs2285053, rs182052, rs6058017, rs1801133, rs832 6, rs1799750, rs2246416, rs4065, rs3829251, rs1667255, r s1137101 |

### [Table 25]

**Table 6: Skin color 3 (skin brightness)**

| | |
|---|---|
| Skin06_linear_m1m2 | rs74653330, rs1800414, rs3785079, rs12785878, rs2234693 , rs1126643, rs2241145, rs6058017, rs1137101, rs1030868 , rs1501299, rs1047781, rs2246416, rs1540771, rs17577, r s1667255, rs3760776, rs6810075, rs1256062, rs7201, rs750 1331, rs2287074, rs182052, rs8326, rs1107946, rs12051272 , rs1801133, rs41281112, rs2108622 |
| Skin06_upper_m1m2 | rs74653330, rs1800414, rs6810075, rs8326, rs1256062, rs1 2785878, rs2285053, rs2282679, rs3760776, rs41281112, rs 1540771, rs3785079, rs2234693, rs2046571, rs2241145, rs2 227564, rs1137101, rs2987983, rs833061, rs1030868, rs150 1299, rs1107946, rs7201, rs1667255, rs964184, rs4065, rs 17577, rs3829251, rs1126643, rs10882272, rs1485766, rs22 98585, rs182052, rs1799724, rs10741657, rs2246416, rs123 77462, rs3865188, rs2232228, rs1047781, rs6058017, rs120 51272, rs1050450, rs10515552 |
| Skin06_lower_m1m2 | rs1800414, rs74653330, rs6058017, rs12785878, rs2246416 , rs1137101, rs17577, rs2234693, rs1485766, rs1061622, r s3785079, rs10515552, rs3829251, rs2228479, rs41303970, rs1047781, rs2241145, rs7501331, rs2285053, rs4654748, r s2287076, rs1030868, rs1126643, rs11234027, rs1256062, r s2108622, rs2232228, rs6810075, rs1540771, rs833061, rs4 880, rs4065 |
| [0087] | |

### [Table 26]

**Table 7: Skin color 4 (yellowness)**

| | |
|---|---|
| Skin07_linear_m1m2 | rs2287074, rs74653330, rs1800414, rs11057830, rs2060793 , rs8326, rs12377462, rs2010963, rs2282679, rs6058017, r s1485766, rs1047781, rs1126643, rs6810075, rs10515552, r s2108622, rs11234027, rs10741657, rs2287076, rs2285053, rs3785079, rs2234693, rs1540771, rs4654748, rs2241145, r s1050565, rs182052, rs1799750, rs9340799, rs4880, rs1256 062, rs1993116, rs3829251, rs1667255, rs2227564, rs22322 28 |
| Skin07_upper_m1m2 | rs2287076, rs11057830, rs2287074, rs74653330, rs2010963 , rs6058017, rs2234693, rs2241145, rs2282679, rs1256062 , rs2060793, rs12377462, rs11234027, rs833061, rs1485766 , rs2108622, rs4654748, rs2228479, rs8110862, rs10515552 , rs1501299, rs1030868, rs10741657, rs7201, rs1800414, r s3760776, rs1107946, rs8326, rsl126643, rs182052, rs7799 039, rs6810075, rs3785079, rs1799750, rs2285053, rs96418 4, rs1667255, rs4065, rs41281112, rs2227564, rs1801133, rs2232228 |
| Skin07_lower_m1m2 | rs8326, rs2287074, rs74653330, rs1256062, rs1047781, rs1 799750, rs3785079, rs1800414, rs182052, rs2285053, rs228 2679, rs6810075, rs12051272, rs2108622, rs1485766, rs222 8479, rs1050565, rs2232228, rs2010963, rs12377462, rs222 7564, rs1126643, rs2060793, rs1667255, rs10515552, rs105 0450, rs10741657, rs1540771, rs2287076, rs7799039, rs720 1, rs17822931, rs3865188 |

### [Table 27]

**Table 8: Wrinkles 1 (Visia wrinkle index)**

| | |
|---|---|
| Skin08_linear_m1m2 | rs7799039, rs2232228, rs12377462, rs1256062, rs3760776, rs6810075, rs1061622, rs2234693, rs1107946, rs12785878, rs2228479, rs6058017, rs2246416, rs1667255, rs1799724, r s1030868, rs182052, rs4654748, rs1126643, rs1799750, rs1 501299, rs2108622, rs11234027, rs2287076, rs17822931, rs 2298585, rs8326, rs1485766, rs2987983, rs11057830, rs488 0, rs1801133, rs7201, rs2227564, rs10741657, rs41281112 , rs964184, rs7501331, rs2282679, rs12051272, rs3865188 , rs10515552, rs833061, rs1540771, rs1800414, rs41303970 , rs1137101, rs2046571, rs9340799, rs1047781 |
| Skin08_upper_m1m2 | rs1107946, rs7799039, rs10741657, rs1501299, rs1126643, rs2232228, rs3760776, rs12051272, rs2046571, rs1799750, rs8110862, rs12377462, rs2282679, rs1801133, rs11234027 , rs2227564, rs1061622, rs2246416, rs1993116, rs41303970 , rs2987983, rs11057830, rs833061, rs2228479, rs3829251 , rs2060793, rs4654748, rs2298585, rs1030868, rs2287076 , rs1667255, rs1800414, rs1540771, rs2285053, rs1050450 , rs964184, rs2234693, rs2241145, rs1799724, rs7201, rs1 047781, rs2287074 |
| Skin08_lower_m1m2 | rs964184, rs2232228, rs1107946, rs1256062, rs7799039, rs 1799724, rs833061, rs12377462, rs6058017, rs2987983, rs7 201, rs2046571, rs182052, rs6810075, rs4654748, rs127858 78, rs2010963, rs41281112, rs2298585, rs2234693, rs11057 830, rs1137101, rs3865188, rs17822931, rs2285053, rs1667 255, rs3785079, rs8326, rs1799750, rs1540771, rs2246416 , rs1061622, rs1047781, rs2287076, rs2227564, rs1030868 , rs1126643, rs3829251, rs8110862, rs1800414, rs1501299 |

### [Table 28]

**Table 9: Wrinkles 2 (Visia wrinkle index)**

| | |
|---|---|
| Skin09_linear_m1m2 | rs2108622, rs1256062, rs7799039, rs3760776, rs1799750, rs 6058017, rs12785878, rs2246416, rs6810075, rs2287076, rs1 107946, rs2228479, rs1540771, rs182052, rs11057830, rs123 77462, rs2232228, rs1799724, rs1667255, rs17822931, rs148 5766, rs1501299, rs1126643, rs1801133, rs4654748, rs18004 14, rs2987983, rs4880, rs3865188, rs2227564, rs7501331, r s8326, rs9340799, rs1030868, rs3829251, rs2046571, rs2298 585, rs10515552, rs11234027, rs8110862, rs1050565, rs1061 622, rs964184, rs10741657, rs7201, rs41281112, rs12934922 , rs1993116, rs3785079, rs1047781, rs17577, rs2282679 |
| Skin09_upper_m1m2 | rs1799750, rs2246416, rs3785079, rs1107946, rs10741657, r s1501299, rs1993116, rs7799039, rs1801133, rs3760776, rs2 046571, rs11234027, rs1667255, rs8110862, rs10882272, rs8 33061, rs2232228, rs9340799, rs2108622, rs4654748, rs2227 564, rs1126643, rs41303970, rs1050450, rs1030868, rs18004 14, rs74653330, rs2060793, rs2234693, rs2228479, rs298798 3, rs1256062, rs12785878, rs2285053, rs1050565, rs7201, r s11057830, rs1540771, rs2241145, rs2010963 |
| Skin09_lower_m1m2 | rs3865188, rs1799724, rs7799039, rs964184, rs12785878, rs 7201, rs3829251, rs6058017, rs1256062, rs2232228, rs12051 272, rs12377462, rs2234693, rs1126643, rs2298585, rs22464 16, rs2287076, rs2046571, rs1107946, rs1800414, rs1485766 , r841281112, rs1047781, rs8326, rs17822931, rs11057830, rs2987983, rs833061, rs1540771, rs6810075, rs2227564, rs3 785079, rs2010963, rs182052, rs1799750 |

### [Table 29]

**Table 10: Texture**

| | |
|---|---|
| Skin10_linear_m1m2 | rs3785079, rs12377462, rs1256062, rs2232228, rs1107946, rs11057830, rs41303970, rs12785878, rs1485766, rs2241145 , rs3829251, rs2227564, rs8326, rs2282679, rs833061, rs1 1234027, rs1047781, rs4654748, rs6058017, rs8110862, rs1 540771, rs2010963, rs3865188, rs2246416, rs2298585, rs16 67255, rs1801133, rs4065, rs7799039, rs1799724, rs103086 8, rs12051272, rs2228479, rs2287074, rs41281112, rs10741 657, rs1993116, rs2285053, rs2108622, rs2060793, rs37607 76 |
| Skin10_upper_m1m2 | rs1107946, rs1137101, rs4065, rs2241145, rs1256062, rs12 934922, rs1667255, rs8326, rs964184, rs3785079, rs412811 12, rs2227564, rs2287074, rs2282679, rs2228479, rs382925 1, rs2232228, rs41303970, rs2046571, rs4654748, rs746533 30, rs7201, rs10515552, rs8110862, rs1801133, rs2285053 , rs2287076, rs2234693, rs10741657, rs2060793, rs1501299 , rs1540771, rs7501331, rs12785878, rs11234027, rs179975 0, rs11057830, rs3865188, rs1030868, rs2108622 |
| Skin10_lower_m1m2 | rs2298585, rs964184, rs3785079, rs1126643. rs12785878, r s1047781, rs833061, rs1667255, rs3760776, rs1256062, rs4 654748, rs12377462, rs2287076, rs2060793, rs3829251, rs1 7822931, rs2108622, rs2987983, rs10741657, rs8110862, rs 41303970, rs2010963, rs2246416, rs11234027, rs1501299, r s4880, rs2234693, rs1061622, rs6058017, rs7501331, rs223 2228 |

### [Table 30]

**Table 11: Elasticity**

| | |
|---|---|
| Skin11_linear_m1m2 | rs3829251, rs11234027, rs1030868, rs964184, rs1050450, r s2232228, rs4654748, rs1485766, rs1061622, rs2287074, rs 1800414, rs1107946, rs2227564, rs3785079, rs7201, rs3760 776, rs10515552, rs2987983, rs1540771, rs10741657, rs406 5, rs1501299, rs2241145, rs7799039, rs10882272, rs746533 30, rs2282679, rs12785878, rs1126643, rs7501331, rs12560 62, rs2108622, rs2246416, rs4880, rs2228479, rs2234693, rs2298585, rs1993116, rs1050565, rs12934922, rs3865188 |
| Skin11_upper_m1m2 | rs1107946, rs10515552, rs1800414, rs964184, rs1485766, r s7201, rs10741657, rs11234027, rs1501299, rs2246416, rs3 865188, rs6810075, rs2010963, rs1540771, rs1030868 |
| Skin11_lower_m1m2 | rs3760776, rs4880, rs1030868, rs1485766, rs1050450, rs20 10963, rs3829251, rs2108622, rs2227564, rs2232228, rs105 0565, rs964184, rs2234693, rs17577, rs1800414, rs1088227 2, rs11234027, rs2987983, rs74653330, rs2298585, rs12785 878, rs1107946, rs12377462, rs12051272, rs1501299, rs750 1331, rs10741657, rs41281112, rs2241145, rs3785079, rs72 01, rs1126643, rs1047781, rs2282679, rs2246416, r54065, rs2285053, rs3865188, rs1061622 |

### [Table 31]

**Table 12: Sebum amount**

| | |
|---|---|
| Skin12_linear_m1m2 | rs1256062, rs74653330, rs2987983, rs1137101, rs3829251, rs6058017, rs2246416, rs17577, rs2234693, rs2046571, rs1 540771, rs4654748, rs3785079, rs2285053, rs2108622, rs81 10862, rs1667255, rs1030868, rs1126643, rs182052, rs1278 5878, rs833061, rs3865188, rs4065, rs1047781, rs10515552 , rs2287076, rs2228479, rs964184, rs2282679, rs6810075, rs1799750, rs11234027, rs1107946, rs9340799, rs7201, rs2 298585, rs2010963, rs12377462, rs2232228, rs1501299, rs1 1057830 |
| Skin12_upper_m1m2 | rs2246416, rs2046571, rs3829251, rs1256062, rs11234027, rs6058017, rs4880, rs1800414, rs1050565, rs1540771, rs10 515552, rs4654748, rs964184, rs1799750, rs1126643, rs148 5766, rs4065, rs10741657, rs2060793, rs2282679, rs228505 3, rs3785079, rs2232228, rs2108622, rs11057830, rs811086 2, rs3865188, rs41303970, rs2987983, rs1030868, rs376077 6, rs12934922, rs1047781, rs17577, rs2234693, rs12377462 , rs1993116, rs833061, rs2227564, rs2287076, rs74653330 , rs1501299 |
| Skin12_lower_m1m2 | rs1256062, rs11057830, rs4880, rs8110862, rs1540771, rs2 108622, rs1047781, rs6058017, rs74653330, rs833061, rs20 10963, rs9340799, rs2287076, rs1801133, rs1501299, rs110 7946, rs2234693, rs2285053, rs7201, rs17577, rs4065 |

### [Table 32]

**Table 13: Moisture content 1, cheek**

| | |
|---|---|
| Skin13_linear_m1m2 | rs1801133, rs964184, rs2246416, rs2298585, rs1667255, rs 74653330, rs1126643, rs1540771, rs8110862. rs2282679, rs 2060793, rs12785878, rs1047781, rs3760776, rs7201, rs204 6571, rs1799750, rs1050565, rs11234027, rs2108622, rs222 7564, rs1485766, rs182052, rs1993116, rs2232228, rs12377 462, rs2287076, rs41281112, rs2241145, rs2987983, rs1074 1657, rs2010963, rs2285053, rs12051272, rs1137101, rs681 0075, rs17822931, rs2228479, rs1501299 |
| Skin13_upper_m1m2 | rs2108622, rs1667255, rs964184, rs7799039, rs1540771, rs 2298585, rs1047781, rs2246416, rs2285053, rs3829251, rs1 2785878, rs10741657, rs2987983, rs1137101, rs2228479, rs 4654748, rs6810075, rs1485766, rs2287076, rs1799750, rs9 340799, rs1801133, rs8110862, rs17822931, rs4880, rs1293 4922, rs1050565, rs3865188, rs2060793, rs1993116, rs1205 1272, rs41281112, rs1107946 |
| Skin13_lower_m1m2 | rs8110862, rs2234693, rs1800414, rs7201, rs2010963, rs12 377462, rs964184, rs1799724, rs2246416, rs74653330, rs11 234027, rs17822931, rs1799750, rs2046571, rs1485766, rs1 1057830, rs1050450, rs10741657, rs1126643, rs833061, rs1 2785878, rs1050565, rs2227564, rs8326, rs2232228, rs1051 5552, rs1667255, rs4065 |

### [Table 33]

**Table 14: Moisture content 2, inner upper arm**

| | |
|---|---|
| Skin14_linear_m1m2 | rs1799724, rs1799750, rs12377462, rs2241145, rs3785079, rs1050565, rs2282679, rs964184, rs1050450, rs1667255, rs 8326, rs17822931, rs74653330, rs2246416, rs9340799, rs11 234027, rs3829251, rs1047781, rs1800414, rs1501299, rs72 01, rs1107946, rs1801133, rs2227564, rs4065, rs2046571, rs833061, rs2010963, rs2285053, rs2298585, rs12785878, r s4880, rs1540771, rs7501331 |
| Skin14_upper_m1m2 | rs1050450, rs2046571, rs1799724, rs12377462, rs3785079, rs964184, rs2241145, rs4880, rs10741657, rs1047781, rs46 54748, rs2108622, rs3865188, rs4065, rs1485766, rs112340 27, rs17822931, rs1126643, rs1050565, rs1667255, rs22826 79, rs12785878, rs3829251, rs1799750, rs1801133, rs29879 83, rs12934922, rs182052, rs2232228, rs41281112, rs11079 46, rs2228479, rs9340799, rs41303970, rs1540771, rs12560 62 |
| Skin14_lower_m1m2 | rs12785878, rs12934922, rs2227564, rs1667255, rs1256062 , rs3785079, rs1047781, rs12377462, rs1050565, rs4880, r s1107946, rs2241145, rs7501331, rs11234027, rs3829251, r s1126643, rs2046571, rs1799750, rs7201, rs4654748, rs964 184, rs1800414, rs1801133, rs2108622 |

### [Table 34]

**Table 15: Barrier, cheek**

| | |
|---|---|
| Skin16_linear_m1m2 | rs1107946, rs2287076, rs41281112, rs3865188, rs2046571, rs1540771, rs2287074, rs1030868, rs1485766, rs1667255, r s1801133, rs7799039, rs2246416, rs1061622, rs10882272, r s833061, rs964184, rs2108622, rs9340799, rs12051272, rs2 227564, rs2228479, rs6810075, rs3829251, rs10741657, rs2 060793, rs4065, rs1799750, rs2232228, rs2234693, rs81108 62, rs2241145, rs2285053, rs2282679, rs2298585, rs7201, rs1256062, rs1799724, rs1993116, rs12785878, rs3760776, rs1800414 |
| Skin15_upper_m1m2 | rs1050565, rs1540771, rs1107946. rs2298585, rs3785079, r s1667255, rs2232228, rs1485766, rs2046571, rs41303970, r s10882272, rs12051272, rs3865188, rs12785878, rs964184, rs2246416, rs1799750, rs2010963, rs17577, rs12377462, rs 2228479, rs2287076, rs2287074, rs1800414, rs2060793, rs4 880, rs1801133, rs7201, rs2241145, rs2285053, rs6810075 , rs1799724, rs10741657, rs1061622, rs9340799, rs2227564 |
| Skin15_lower_m1m2 | rs7799039, rs2228479, rs2285053, rs964184, rs2246416, rs 1501299, rs7501331, rs2287076, rs2282679, rs8110862, rs2 287074, rs1667255, rs11057830, rs833061, rs1485766, rs18 2052, rs4654748, rs7201, rs2241145, rs41281112, rs123774 62, rs12785878, rs2046571, rs11234027, rs3829251, rs3865 188, rs4065, rs2227564, rs41303970 |

### [Table 35]

**Table 16: Barrier, inner upper arm**

| | |
|---|---|
| Skin16_linear_m1m2 | rs2285053, rs833061, rs2282679, rs2298585, rs2010963, rs 1799724, rs1061622, rs964184, rs2287076, rs2108622, rs16 67255, rs1030868, rs4654748, rs1540771, rs1799750, rs224 6416, rs2241145, rs2232228, rs3865188, rs2287074, rs7501 331, rs11057830, rs2987983, rs12785878, rs1126643, rs746 53330, rs9340799, rs182052, rs3785079, rs2227564, rs1074 1657, rs1501299, rs2234693, rs12934922, rs12377462, rs18 01133, rs7799039, rs6810075, rs8326, rs1047781, rs17577 |
| Skin16_upper_m1m2 | rs2285053, rs1030868, rs2241145, rs12377462, rs2010963, rs2234693, rs1799750, rs1667255, rs2287074, rs2287076, r s1540771, rs8326, rs3865188, rs1126643, rs10882272, rs20 46571, rs182052, rs6810075, rs833061, rs1061622, rs17997 24, rs2282679, rs41303970, rs3785079, rs1050450, rs10477 81, rs12051272, rs8110862, rs1256062, rs12785878, rs9641 84, rs10741657, rs2232228 |
| Skin16_lower_m1m2 | rs2282679, rs2010963, rs12785878, rs833061, rs17577, rs2 228479, rs1107946, rs1256062, rs1540771, rs964184, rs106 1622, rs2246416, rs2232228, rs17822931, rs1501299, rs378 5079, rs74653330, rs4654748, rs2108622. rs1137101, rs107 41657, rs41303970, rs2227564, rs8110862, rs1667255 |

### [Table 36]

**Table 17: Wrinkle area**

| | |
|---|---|
| Skin21_linear_m1m2 | rs74653330, rs2298585, rs1061622, rs2282679, rs2108622, rs1540771, rs11057830, rs833061, rs1799724, rs2287074, r s2232228, rs3760776, rs1993116, rs7201, rs964184, rs1074 1657, rs12051272, rs2287076, rs12785878, rs1799750, rs16 67255, rs8326, rs1800414, rs12377462, rs1256062, rs14857 66, rs2285053, rs2228479, rs6810075, rs182052, rs1107946 , rs2246416, rs1501299, rs3829251, rs11234027, rs4880 |
| Skin21_upper_m1m2 | rs2108622, rs1540771, rs7201, rs1993116, rs2046571, rs22 85053, rs2298585, rs10741657, rs1801133, rs12377462, rs1 2785878, rs3785079, rs2227564, rs12934922, rs1050450, rs 1256062, rs17577, rs6810075, rs182052, rs1667255, rs1205 1272, rs4654748, rs11234027, rs2987983, rs964184, rs2287 076, rs3829251, rs1799750, rs2232228 |
| Skin21_lower_m1m2 | rs1540771, rs1799724, rs3760776, rs11057830, rs10741657 , rs2010963, rs182052, rs1061622, rs1256062, rs2987983, rs7201, rs3865188, rs74653330, rs1107946, rs6810075, rs2 246416, rs833061, rs2108622, rs2287074, rs1050450, rs123 77462, rs12785878, rs1126643, rs2298585, rs1993116, rs18 00414, rs2241145, rs2228479, rs3785079, rs2282679, rs222 7564, rs41281112, rs1485766, rs4065, rs1501299 |

### [Table 37]

**Table 18: Wrinkle volume**

| | |
|---|---|
| Skin22_linear_m1m2 | rs2298585, rs74653330, rs1061622, rs1540771, rs2282679, rs3785079, rs833061, rs2232228, rs2108622, rs11057830, r s1799724, rs3760776, rs1799750, rs1256062, rs182052, rs3 829251, rs964184, rs7201, rs2287074, rs8326, rs11234027 , rs2287076, rs10515552. rs12377462, rs1667255, rs120512 72, rs2285053, rs1485766, rs8110862, rs1993116, rs681007 5, rs2987983, rs12785878, rs10741657, rs2246416, rs11079 46, rs2010963, rs2228479, rs1800414 |
| Skin22_upper_m1m2 | rs1540771, rs3785079, rs2010963, rs2108622, rs7201, rs83 26, rs833061, rs1993116, rs2298585, rs1107946, rs1074165 7, rs1801133, rs964184, rs1799724, rs182052, rs1061622, rs11057830, rs41303970, rs1800414, rs2287074, rs2287076 , rs3829251, rs6810075, rs2241145, rs2232228, rs1501299 , rs4654748, rs12785878, rs11234027, rs12051272 |
| Skin22_lower_m1m2 | rs1540771, rs2298585, rs1799724, rs2227564, rs1061622, r s12785878, rs2246416, rs2282679, rs2010963, rs2108622, r s11057830, rs1256062, rs833061, rs2241145, rs182052, rs1 050450, rs3865188, rs6810075, rs1485766, rs7201, rs18004 14, rs3760776, rs1126643, rs2232228, rs2987983, rs779903 9, rs1137101, rs1107946, rs17822931, rs964184, rs1074165 7, rs9340799, rs1993116, rs8110862, rs12051272, rs223469 3, rs8326 |

### [Table 38]

**Table 19: Maximum wrinkle depth**

| | |
|---|---|
| Skin23_linear_m1m2 | rs1667255, rs10515552, rs1801133, rs2108622, rs833061, r s11057830, rs3785079, rs964184, rs2010963, rs1107946, rs 4654748, rs2287076, rs2285053, rs1485766, rs1800414, rs1 799724, rs2241145, rs2298585, rs3760776, rs8326, rs10616 22, rs1126643, rs10741657, rs1540771, rs2287074, rs41281 112, rs2282679, rs74653330, rs3829251, rs1050565, rs1047 781, rs7201, rs12377462, rs182052, rs11234027, rs2228479 , rs1137101, rs17577, rs2227564, rs6058017, rs2987983, r s1256062, rs2046571 |
| Skin23_upper_m1m2 | rs1800414, rs1107946, rs1137101, rs2227564, rs2108622, r s7201, rs1801133, rs11057830, rs964184, rs8326, rs378507 9, rs2285053, rs1485766, rs1256062, rs1061622, rs1051555 2, rs2241145, rs1667255, rs2228479, rs3760776, rs1540771 , rs1126643, rs12377462, rs833061, rs41303970. rs3865188 , rs8110862, rs2987983, rs10741657, rs2287074, rs1799750 , rs2246416, rs1501299, rs10882272 |
| Skin23_lower_m1m2 | rs1540771, rs182052, rs2287074, rs833061, rs2046571, rs2 287076, rs2228479, rs1799724, rs1061622, rs41281112, rs9 64184, rs2232228, rs2298585, rs12377462, rs9340799, rs29 87983, rs1050450, rs8326, rs7201, rs2234693, rs1801133, rs6810075, rs1107946, rs1126643, rs2282679, rs12934922, rs3785079, rs1800414, rs2241145, rs3760776, rs3865188, r s4880, rs11057830, rs1047781, rs8110862, rs3829251 |

An ROC curve was obtained for the created learning models for assessing skin type, based on tendency for (or tendency against) a skin type outputted by inputting an explanatory variable of validation data, and classification into the high value group (or low value group) for the skin condition of the validation data, and the area under the ROC curve (AUC) and sensitivity (Sensitivity) were calculated (Fig. 6A). The sensitivity was defined as the optimum cutoff value calculated from the ROC curve (the value of the point where the distance from the upper left corner of the ROC curve (the point where both the specific sensitivity and specificity were 1) was minimal), or a value set independently from the ROC curve (for example, the cutoff value was set to 0.5 since the skin type score is a probability value obtained from a predicted model). SNP appearance frequency was examined for the 100 different learning models for determining skin types which were created in this manner (Fig. 6C). SNPs to be used in the learning model were selected based on the SNP appearance frequency and biochemical findings, and learning models with high AUC were selected from among the created learning models, using the SNPs (Fig. 5B).

Coefficients of determination (R²) were calculated for the created learning models for determining skin condition score, from skin condition scores outputted by inputting explanatory variables of validation data, and the measured values for the skin condition in the validation data (Fig. 6B). SNP appearance frequency was examined for the 100 different learning models which were created in this manner (Fig. 6C). SNPs to be used in the learning model were selected based on the SNP appearance frequency and biochemical findings, and learning models with high R² were selected from among the created learning models, using the SNPs (Fig. 5A).

SNPs contributing to the skin condition were selected by this procedure. SNPs selected with measured values for a skin condition as response variables are displayed as "Linear". SNPs selected with classification results into high value groups as response variables are displayed as "upper". SNPs selected with classification results into low value groups as response variables are displayed as "lower". Both SNPs are listed, for when the number of SNPs used was 1 to 4 and when it was 5 to 10. The skin conditions represented as codes: Skin 01 to 16 and Skin 21 to 23 are shown in the following table.

**[Table 39]**

| Code | Skin condition | Code | Skin condition |
|---|---|---|---|
| Skin 01 | Pigmented spots 1 | Skin 11 | Elasticity |
| Skin 02 | Pigmented spots 2 | Skin 12 | Sebum amount |
| Skin 03 | Pigmented spots 3 | Skin 13 | Moisture content 1 |
| Skin 04 | Skin color 1 | Skin 14 | Moisture content 2 |
| Skin 05 | Skin color 2 | Skin 15 | Barrier 1 |
| Skin 06 | Skin color 3 | Skin 16 | Barrier 2 |
| Skin 07 | Skin color 4 | Skin 21 | Wrinkle area |
| Skin 08 | Wrinkles 1 | Skin 22 | Wrinkle volume |
| Skin 09 | Wrinkles 2 | Skin 23 | Maximum wrinkle depth |
| Skin 10 | Texture | | |

### Example 7: Method of evaluating skin condition using created learning models for determining skin condition score

The skin conditions of test subjects were evaluated using a learning unit containing the learning model for determining skin condition score created in Example 4 and validated in Example 6. Specifically, SNP-related genetic information and environmental factor information selected from the group consisting of age, body height, body weight, BMI, ultraviolet exposure information and smoking information were inputted as explanatory variables into a learning unit created to determine a score for a skin condition selected from the group consisting of pigmented spots 1 to 3, skin colors 1 to 4, wrinkles 1 and 2, texture, elasticity, sebum amount, moisture contents 1 and 2, barrier functions 1 and 2, wrinkle area, wrinkle volume and maximum wrinkle depth. Upon input of genetic information and environmental information as explanatory variables, the learning unit outputted a score for congenital skin characteristics according to the formula of the learning model. Upon changing age or behavioral factors in the formula of the learning model, the change in future skin condition was predicted. By selecting tanning countermeasures and smoking behavior as behavioral factors, it was possible to predict a score of skin condition for different ages (Figs. 9A and B).

### Example 8: Assessment of skin type using created learning model for assessing skin type

The skin conditions of test subjects were evaluated using a learning unit containing the learning model for assessing skin type created in Example 5 and validated in Example 6. Specifically, SNP-related genetic information and environmental factor information selected from the group consisting of age, body height, body weight, BMI, ultraviolet exposure information and smoking information were inputted into a learning unit created to determine a score for a skin condition selected from the group consisting of pigmented spots 1 to 3, skin colors 1 to 4, wrinkles 1 and 2, texture, elasticity, sebum amount, moisture contents 1 and 2, barrier functions 1 and 2, wrinkle area, wrinkle volume and wrinkle maximum depth. Upon inputting explanatory variables related to genetic information and environmental information, the learning unit outputted the tendency for a skin type (possibility of assignment to the high value group of the skin condition) and/or tendency against the skin type (possibility of assignment to the low value group of the skin condition), as response variables, according to the formula of the learning model. The outputted tendency for the skin type (possibility of assignment to the high value group of the skin condition) and/or tendency against the skin type (possibility of assignment to the low value group of the skin condition) were each represented as a plot diagram and sector graph (Fig. 10A, Fig. 10B).

### REFERENCE SIGNS LIST

10 Information processing device
11 Storage unit
12 Input unit
13 Learning unit
14 Processing unit
15 Output unit
20 Evaluation system
30 Learning device

## Claims

1. A skin evaluating method using individual SNP information and at least one environmental factor information selected from the group consisting of age, body height, body weight, BMI, ultraviolet exposure information and smoking information,
wherein SNP information and environmental factor information are weighted for contribution to skin condition, to determine the skin condition.

2. The skin evaluating method according to claim 1, wherein the weighting is determined by multiple regression analysis using SNP information and environmental factor information as explanatory variables and measured values for a skin condition as response variables.

3. The skin evaluating method according to claim 2, wherein age is used as a variable, and future skin condition is predicted based on a graph representing the relationship with a skin condition score.

4. The skin evaluating method according to claim 3, wherein the future skin condition is predicted for change in a behavioral factor among the environmental factors.

5. The skin evaluating method according to claim 4, wherein the behavioral factors are countermeasures against ultraviolet rays and smoking habit.

6. The skin evaluating method according to claim 1, wherein the weighting is determined by logistic regression from SNP information and environmental factor information for a test subject, with division into a high value group and a non-high value group that have been determined based on measured values for a skin condition, and using the classification results into the high value group and non-high value group as response variables.

7. The skin evaluating method according to claim 1 or 6, wherein the weighting is determined by logistic regression from SNP information and environmental factor information for a test subject, with division into a low value group and a non-low value group that have been determined based on measured values for a skin condition, and using the classification results into the low value group and non-low value group as response variables.

8. A skin evaluation system comprising a processing unit (14), an input unit (12), a learning unit (13) and output unit (15), wherein the skin evaluation system includes the following:
an input unit (12) from which individual SNP information, and environmental factor information selected from the group consisting of age, body height, body weight, BMI, ultraviolet exposure information and smoking information, are inputted,
a learning unit (13) which uses a learning model which has learned based on the relationship of SNP information and environmental factor information with skin condition, and which is weighted for contribution to skin condition, to determine information regarding skin condition,
a processing unit (14) that processes the skin condition that has been determined by the learning unit (13), and
an output unit (15) that outputs the processed skin condition.

9. The evaluation system according to claim 8, wherein the learning unit (13) includes a learning model having SNP information and environmental factor information as explanatory variables and measured values for a skin condition as response variables, and wherein each of the explanatory variables are weighted against the response variables by multiple regression analysis.

10. The evaluation system according to claim 9, which comprises:
the learning unit (13) determining a skin condition and determining a formula with age as a variable, as information for the skin condition,
the processing unit (14) generating a graph of change in the skin condition with respect to age, based on the formula, and
the output unit (15) outputting a graph of change in skin condition with respect to age, as the processed skin condition.

11. The evaluation system according to claim 9, which comprises:
the learning unit (13) determining a skin condition and outputting a formula with age and a behavioral factor as variables, as information for the skin condition,
the processing unit (14) generating a graph of change in skin condition score with respect to age for change in the behavioral factor, based on the formula, and
the output unit (15) outputting a graph of change in skin condition with respect to age, as the processed skin condition.

12. The evaluation system according to claim 11, wherein the behavioral factors are countermeasures against ultraviolet rays and smoking habit.

13. The evaluation system according to claim 8, wherein the learning unit (13) is weighted by logistic regression from SNP information and environmental factor information for a test subject, with division into a high value group and a non-high value group that have been determined based on measured values for a skin condition, and using the classification results into the high value group and non-high value group as response variables.

14. The evaluation system according to claim 8 or 13, wherein the learning unit (13) is weighted by logistic regression from SNP information and environmental factor information for a test subject, with division into a low value group and a non-low value group that have been determined based on measured values for a skin condition, and using the classification results into the low value group and non-low value group as response variables.

15. A program for controlling a skin evaluation system comprising a learning unit (13) that has been trained based on the relationship between SNP information, environmental factor information and a skin condition, and that determines the skin condition via a learning model that has been weighted for contribution to the skin condition, an input unit (12), an output unit (15) and a processing unit (14), the program including the following commands:
inputting individual SNP information and environmental factor information selected from the group consisting of age, body height, body weight, BMI, ultraviolet exposure information and smoking information, which has been inputted through the input unit (12), to the learning unit (13),
setting information for a determined skin condition via a learning model for inputted SNP information and environmental factor information which has been weighted for contribution to skin type, at the learning unit (13),
processing information for the set skin condition to the processing unit (14), and
outputting the processed skin condition to the output unit (15).

16. The program according to claim 15, wherein the learning unit (13) includes a learning model having SNP information and environmental factor information as explanatory variables and measured values for a skin condition as response variables, and wherein each of the explanatory variables are weighted against the response variables by multiple regression analysis.

17. The program according to claim 15, which includes a command that:
causes the learning unit (13) to determine a skin condition and determine a formula with age as a variable, as information for the skin condition,
causes the processing unit (14) to generate a graph of change in the skin condition with respect to age, based on the formula, and
causes the output unit (15) to output a graph of change in skin condition with respect to age, as the processed skin condition.

18. The program according to claim 15, which includes a command that:
causes the learning unit (13) to determine a skin condition and output a formula with age and a behavioral factor as variables, as information for the skin condition,
causes the processing unit (14) to generate a graph of change in skin condition score with respect to age for change in the behavioral factor, based on the formula, and
causes the output unit (15) to output a graph of change in skin condition with respect to age, as the processed skin condition.

19. The program according to claim 18, wherein the behavioral factors are countermeasures against ultraviolet rays and smoking habit.

20. The program according to claim 15, wherein the learning unit (13) is weighted by logistic regression from SNP information and environmental factor information for a test subject, with division into a high value group and a non-high value group that have been determined based on measured values for a skin condition, and using the classification results into the high value group and non-high value group as response variables.

21. The program according to claim 15 or 16, wherein the learning unit (13) is weighted by logistic regression from SNP information and environmental factor information for a test subject, with division into a low value group and a non-low value group that have been determined based on measured values for a skin condition, and using the classification results into the low value group and non-low value group as response variables.
